# EUROPEAN PATENT APPLICATION

(11) **EP 4 578 409 A1**
(43) Date of publication of application: **02.07.2025**
(21) Application number: 22956321.8
(22) Date of filing: 02.12.2022
(51) Int. Cl.: A61B 17/34

(54) **APPLICATION APPARATUS FOR APPLYING MEDICAL INSTRUMENT TO HOST**

(30) Priority: 26.08.2022 CN 202211034009
(71) Applicant: Shenzhen Sisensing Co., Ltd., Shenzhen, Guangdong 518110 (CN)
(72) Inventor: LI, Yunfeng, Shenzhen, Guangdong 518110 (CN); WU, Jiang, Shenzhen, Guangdong 518110 (CN); CHEN, Sai, Shenzhen, Guangdong 518110 (CN)
(74) Representative: Isern Patentes y Marcas S.L.
(86) International application number: PCT/CN2022/136108
(87) International publication number: WO 2024/040787

(57) **Abstract**

The present disclosure describes an application apparatus(1000) for applying a medical instrument(800) to a host, including a housing(10), an auxiliary mechanism(20) and a first driving mechanism(30), the housing(10) includes a first holding portion(130), a proximate end which is close to the host during operation and a distal end which is far away from the host; the auxiliary mechanism(20) includes a moving main body(200) releasably held on the first holding portion(130) and configured to move relative to the housing(10) when released, an accommodating portion(250) provided on the moving main body(200) and configured to be coupled with the medical instrument(800), and a piercing member(260) provided on the moving main body(200); the first driving mechanism(30) is configured to apply a force to the moving main body(200) in a mode of facing the proximate end; and the first driving mechanism(30) drives the moving main body(200) to move towards the proximate end when the moving main body(200) is released so as to push the medical instrument(800) which is coupled with the accommodating portion(250) to a body surface of the host and to at least partially place the medical instrument(800) subcutaneously on the host.

## Description

### Background of invention

### Field of Invention

The present invention substantially relates to medical instruments, and particularly relates to an application apparatus for applying a medical instrument to a host.

### Description of Related Art

Due to the need of clinical diagnosis or personal health monitoring, for example, for a diabetic patient, it is very necessary to continuously monitor the glucose concentration of tissue fluid in real time daily, and adjust the glucose concentration in time by means of, for example, adjusting diet or applying drug therapy, thereby reducing and decreasing the occurrence of complications due to abnormal glucose concentration.

At present, a user usually monitors the glucose concentration in the tissue fluid using a glucose sensor that can be inserted inside the skin and react with glucose in the tissue fluid and an electronic apparatus that is applied to the skin and is connected to the glucose sensor. In order to insert the glucose sensor inside the skin and to apply the electronic apparatus to the skin, an insertion apparatus is usually to be with help of. Specifically, the insertion apparatus may include a needle that can pierce the skin, through which the glucose sensor is inserted inside the skin, and then the needle is separated from the glucose sensor and removed from the skin.

However, in the above-mentioned prior art, a process of inserting the needle inside the skin or removing the needle from the skin needs to be driven by means of manpower. In this case, since it is possible that the force of driving the needle, the position of inserting the needle inside the skin, the depth of inserting the needle inside the skin, or the timing of removing the needle from the skin, etc. are difficult to be accurately controlled, on one hand, user's experience when inserting the glucose sensor may be affected, and on the other hand, it is possible that the accuracy of the acquired glucose concentration is not high since the glucose sensor is not inserted into a desired position.

### Summary of invention

The present invention is accomplished in consideration of the above conditions in the prior art, and the objective is to provide an application apparatus which is easy and convenient to operate and can accurately apply a medical instrument to a host.

Therefore, the present invention provides an application apparatus for applying a medical instrument to a host. The application apparatus includes a housing, an auxiliary mechanism and a first driving mechanism, wherein the housing includes a first holding portion, a proximate end which is close to the host during operation and a distal end which is far away from the host, the auxiliary mechanism includes a moving main body which is releasably held on the first holding portion and is configured to move relative to the housing when released, an accommodating portion provided on the moving main body and configured to be combined with the medical instrument, and a piercing member provided on the moving main body, the first driving mechanism is configured to apply a force to the moving main body in a mode of facing the proximate end, and the first driving mechanism drives the moving main body to move towards the proximate end when the moving main body is released so as to push the medical instrument combined with the accommodating portion to a body surface of the host and to place the medical instrument at least partially subcutaneously on the host.

In the application apparatus according to the present embodiment, the housing includes the proximate end which is close to the host and the distal end which is away from the host during operation; the accommodating portion capable of receiving and storing the medical instrument and the piercing member arranged on the moving main body are provided; and the moving main body is driven by the first driving mechanism in the mode of facing the proximate end so that the medical instrument can be at least partially placed inside the skin of the host. In this case, in the medical instrument application process, the driving mechanism is configured to apply a force, thereby enabling to easily and conveniently apply the medical instrument to the host.

In addition, in the application apparatus according to the present embodiment, optionally, the medical instrument includes a sensor partially placed subcutaneously on the host and an application portion coupled with the sensor, the application portion has a first upper body part physically connected with the sensor and applied to the body surface of the host, and a second upper body part electrically connected with the sensor and detachably assembled with the first upper body part. Thereby, it is facilitated to reuse the second upper body part.

In addition, in the application apparatus according to the present embodiment, optionally, the accommodating portion is detachably coupled to the first upper body part.

In addition, in the application apparatus according to the present embodiment, optionally, the piercing member includes a sharp object passing through the accommodating portion and configured to at least partially accommodate the sensor, and a support seat releasably supporting the sharp object. In this case, the sensor can be carried by the sharp object to be placed at least partially subcutaneously on the host.

In addition, in the application apparatus according to the present embodiment, optionally, the application apparatus further includes a disassembling mechanism used for separating the sharp object from the support seat.

In addition, in the application apparatus according to the present embodiment, optionally, the moving main body includes a first locking portion configured to be releasably interlocked with the first holding portion. Therefore, the moving main body can be releasably held on the first holding portion through cooperation of the first locking portion and the first holding portion.

In addition, in the application apparatus related to the present embodiment, optionally, the application apparatus further includes a first triggering mechanism configured to enable the first holding portion to be separated from the first locking portion so as to release the moving main body. Thereby, the moving main body can be conveniently released through the first triggering mechanism.

In addition, in the application apparatus according to the present embodiment, optionally, the auxiliary mechanism further includes a second holding portion provided on the moving main body, and the piercing member is releasably held on the second holding portion and is configured to move relative to the accommodating portion when released.

In addition, in the application apparatus according to the present embodiment, optionally, the piercing member includes a second locking portion configured to be releasably interlocked with the second holding portion. Thereby, the piercing member can be releasably held on the second holding portion through the cooperation of the second locking portion and the second holding portion.

In addition, in the application apparatus according to the present embodiment, optionally, the application apparatus further includes a second driving mechanism, and the second driving mechanism is configured to apply a force to the piercing member in a mode of facing the distal end.

According to the present disclosure, the application apparatus which is easy and convenient to operate and can accurately apply the medical instrument to the host can be provided.

### Brief description of the drawings

FIG. 1 shows an application overview of a medical instrument and an application apparatus according to an example of the present embodiment.
FIG. 2 is a schematic diagram of a first view angle of a medical apparatus according to an example of the present embodiment.
FIG. 3 is a schematic diagram of a second view angle of a medical apparatus according to an example of the present embodiment.
FIG. 4 is a schematic diagram of a first view angle of an application portion in a explode state according to an example of the present embodiment.
FIG. 5 is a schematic diagram of a second view angle of an application portion in a explode state according to an example of the present embodiment.
FIG. 6 is a schematic block diagram of an electronic apparatus of a medical instrument according to an example of the present embodiment.
FIG. 7A is an overall appearance schematic diagram of a first view angle of an application apparatus according to an example of the present embodiment; FIG. 7B is an overall appearance schematic diagram of a second view angle of an application apparatus according to an example of the present embodiment; and FIG. 7C is a explode schematic diagram of an application apparatus according to an example of the present embodiment.
FIG. 8A is a perspective schematic diagram of a peripheral part of a first housing according to an example of the present embodiment; FIG. 8B is a perspective schematic diagram of a second housing according to an example of the present embodiment; and FIG. 8C is a perspective schematic diagram of a moving main body according to an example of the present embodiment.
FIG. 9 is a partial schematic diagram of a first locking portion and a first holding portion when a moving main body is held according to an example of the present embodiment.
FIG. 10 is a partial schematic diagram of a first triggering mechanism according to an example of the present embodiment.
FIG. 11 is a perspective schematic diagram of a moving main body according to an example of the present embodiment.
FIG. 12A is a explode schematic diagram of a first view angle of a second housing and an auxiliary mechanism; FIG. 12B is a explode schematic diagram of a second view angle of a second housing and the auxiliary mechanism; FIG. 12C is a cross-sectional schematic diagram of an auxiliary mechanism assembled to a second housing; FIG. 12D is a schematic diagram of a cover according to an example of the present embodiment.

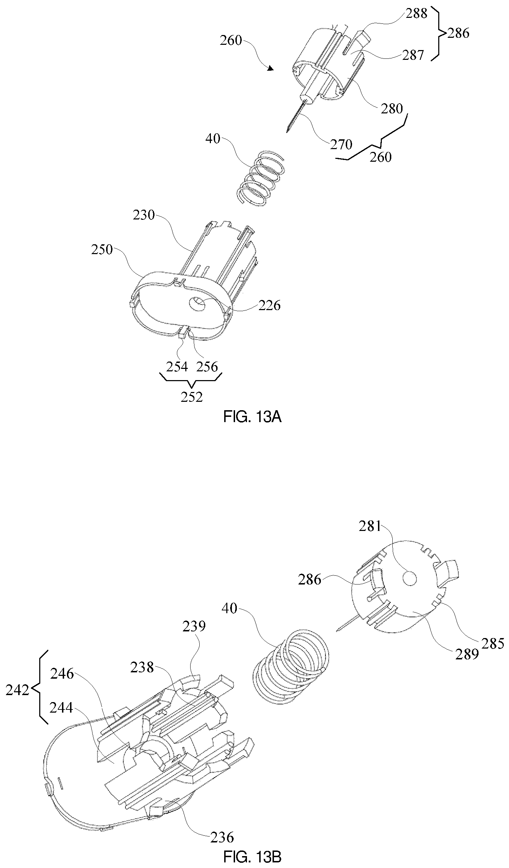

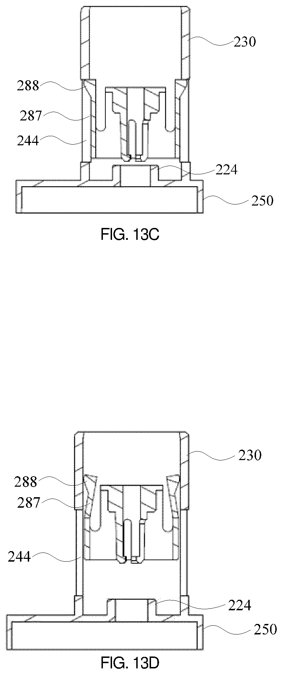

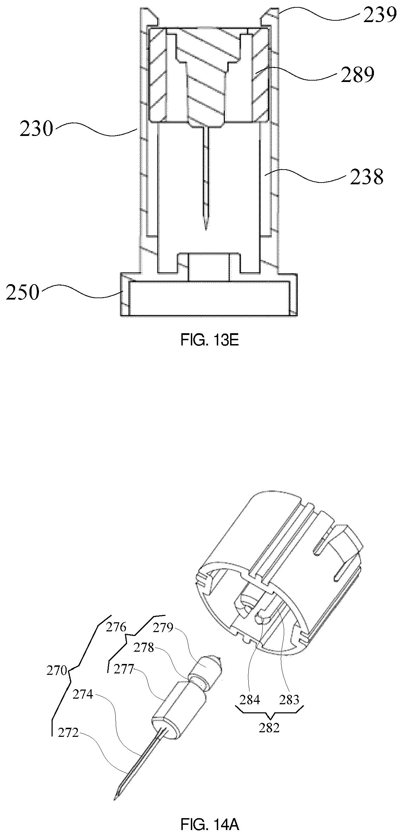

FIG. 13A is a disassembly schematic diagram of a first view angle of a moving main body and a piercing member according to an embodiment; FIG. 13B is a disassembly schematic diagram of a second view angle of a moving main body and a piercing member according to an embodiment; FIG. 13C is a cross-sectional schematic diagram of a held piercing member according to an embodiment; FIG. 13D is a cross-sectional schematic diagram of a released piercing member according to an embodiment; and FIG. 13E is a cross-sectional schematic diagram of a moving main body and a piercing member in pre-assembling according to an embodiment.
FIG. 14A is a explode schematic diagram of a piercing member according to an example of the present embodiment; FIG. 14B is an assembly schematic diagram of a piercing member according to an example of the present embodiment; FIG. 14C is an enlargement schematic diagram of a needle-shaped portion according to an example of the present embodiment; and FIG. 14D is an assembly schematic diagram of a sharp object and a first upper body part according to an example of the present embodiment.
FIG. 15A is a schematic diagram of a sharp object not disassembled; and FIG. 15B is a schematic diagram of a sharp object disassembled. And
FIG. 16 is a perspective schematic diagram of a box apparatus according to an example of the present embodiment.

### Detailed description

Hereinafter, preferred embodiments of the present invention will be described in detail with reference to the accompanying drawings. In the following description, the same components are denoted by the same reference numerals, and the description thereof will not be repeated. In addition, the drawings are merely schematic views, and the ratio of the dimensions of the components to each other or the shape of the components may be different from the actual ones.

It should be noted that the terms "comprising" and "having", and any variants thereof, such as a process, method, system, product, or device, which are included or have a series of steps or units, are not necessarily limited to those steps or units are provided, but may include or have other steps or units not explicitly listed or inherent to such processes, methods, products or devices.

An embodiment of the present disclosure relates to an application apparatus of a medical instrument, the application apparatus can be used to apply the medical apparatus to a host, for example, apply the medical apparatus to a body surface of the host, or to place the medical apparatus entirely or partially subcutaneously on the host. The application apparatus according to this embodiment may facilitate the application of the medical apparatus to a desired location.

The application apparatus according to this embodiment can also be referred to as, for example, a boosting apparatus, a conveying apparatus, an implanting apparatus, an application apparatus and an auxiliary apparatus. It is to be noted that the names are indicative of the application apparatus for applying the medical instrument to the host according to the present embodiment and should not be understood as being restrictive.

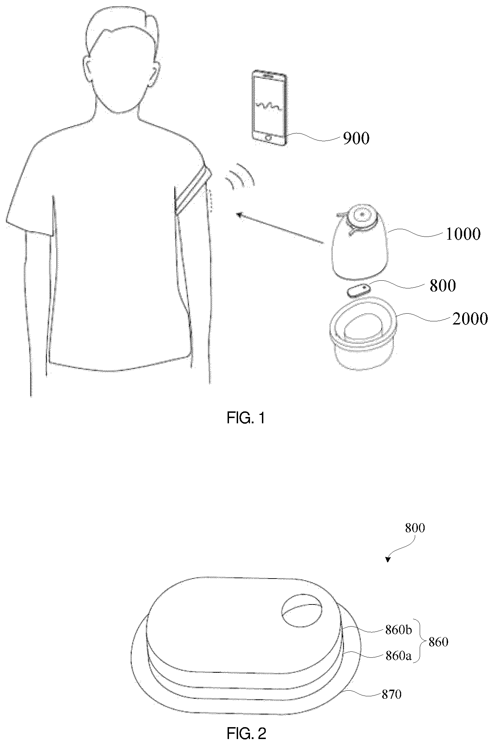

FIG. 1 shows the application overview of the medical instrument 800 and the application apparatus 1000 according to an example of the present embodiment. In this embodiment, the medical instrument 800 can be applied to the host through the application apparatus 1000 and applied to the desired position, and the host can acquire physiological information through the medical instrument 800 applied to the host.

In addition, the present disclosure further provides a monitoring system, the monitoring system may include the medical instrument 800 capable of acquiring the physiological information of the host according to this embodiment, and a reading device 900 (see FIG. 1) in communication connection with the medical instrument 800. The medical instrument 800 applied to the host can transmit the acquired physiological information to the reading apparatus 900 in a wireless mode, and thereby it is facilitated for the host to read and monitor the physiological information.

In addition, the present disclosure also provides a box apparatus 2000 (see FIG. 1), the box apparatus 2000 may be used for fully or partially accommodating the medical apparatus 800 according to this embodiment. In some examples, the medical instrument 800 according to this embodiment can be fully or partially accommodated in the box apparatus 2000 before applied to the host.

In addition, the present disclosure also provides an instrument suite, the instrument suite may include the medical instrument 800 capable of acquiring physiological information of the host according to this embodiment, the application apparatus 1000 capable of applying the medical instrument 800 to the host according to this embodiment, and the box apparatus 2000 capable of accommodating the medical instrument 800 according to the embodiment. The medical instrument 800 can be fully or partially accommodated in the box apparatus 2000 before applied to the host, and when the medical instrument 800 is needed, the medical instrument 800 can be picked up and applied to the host through the application apparatus 1000.

In some examples, the medical instrument 800 may be a sensing apparatus. In some examples, the medical instrument 800 may be an analyte sensor apparatus which may generate information of a specific analyte in body fluid and the like based on the body fluid, for example, the analyte sensor apparatus may react with the analyte in the body fluid and generate analyte information. In this case, the sensor reacts with the analyte in the body fluid, and thereby it is facilitated for the analyte information in the body fluid to be obtained.

In present embodiment, the analyte of the analyte sensor apparatus may be one or more of glucose, acetylcholine, amylase, bilirubin, cholesterol, chorionic gonadotropin, creatine kinase, creatine, creatinine, DNA, fructosamine, glutamine, growth hormone, hormone, ketone bodies, lactate, oxygen, peroxide, prostate-specific antigen, prothrombin, RNA, thyroid stimulating hormone and troponin.

Hereinafter, the medical instrument 800 according to the embodiment is illustrated by taking glucose as an analyte. It is to be noted that for other analytes, those skilled in the art can analyze the other analytes with a slight modification to the medical instrument 800 employed on basis of glucose.

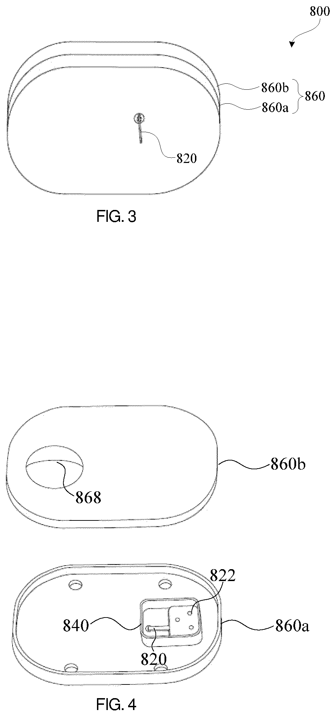

FIG. 2 is the schematic diagram of the first view angle of the medical instrument 800 according to an example of the present embodiment. FIG. 3 is the schematic diagram of the second view angle of the medical instrument 800 according to an example of the present embodiment.

In some examples, the medical instrument 800 may include a sensor 820 and an application portion 860 (see FIGS. 2-3). The sensor 820 may be assembled to the application portion 860.

In consideration of facilitating to react with tissue fluid, for example, the body fluid, the sensor 820 may be partially or entirely placed subcutaneously on the host. The sensor 820 may react with glucose in subcutaneous tissue fluid after placed subcutaneously to generate glucose information of the host. The application portion 860 can be applied to the body surface of the host. An electronic apparatus 880 (described later) provided on the application portion 860 may receive the glucose information generated by the sensor 820. In addition, the electronic apparatus 880 may also provide the sensor 820 with electric power for acquiring the physiological information. Thereby, it is facilitated to perform continuous monitoring of the sensor 820 subcutaneously on the host.

In some embodiments, the sensor 820 may include an implanting part and a connecting part. The implanting part can be placed subcutaneously on the host and connected to the application portion 860 through the connecting part. In this case, by placing the implanting part subcutaneously on the host and connecting the implanting part to the application portion 860 through the connecting part, the temporary fixation of the sensor 820 at a particular location of the host can be facilitated. In some examples, the connecting part can be connected to the application portion 860 via a connecting seat 840 (described later).

In some examples, the implanting part of the sensor 820 may be of elongated shape. In addition, the implanting part may be rigid. Thereby, it is facilitated for the implanting part to be placed subcutaneously on the host. In other examples, the implanting part may also be flexible, in this case, the foreign body feeling of the host can be reduced. In terms of size, the length of the implanting part may be 1 mm to 10 mm, for example, 1 mm, 2 mm, 3 mm, 4 mm, 5 mm, 6 mm, 7 mm, 8 mm, 9 mm, or 10 mm. After the implanting part is implanted in the host with the depth thereof may reaching the dermis layer and location being between tissues of the skin, so that the sensor 820 may collect glucose information of interstitial fluid.

The implanting part may include a working electrode (not shown) and a counter electrode (not shown). In present embodiment, a sensing layer including glucosase may be provided on the working electrode, and the sensor 820 placed subcutaneously may generate a current signal by redox reaction of glucosase on the working electrode with glucose in the tissue fluid or blood and forming a loop with the counter electrode, and the current signal is analyzed to obtain glucose concentration information. The current signal generated by the sensor 820 can be transmitted to the electronic apparatus 880.

In some examples, the implanting part may further include a reference comparison electrode (not shown). The reference comparison electrode can form a known and fixed potential difference with the tissue fluid or blood. In this case, the potential difference between the working electrode and the tissue fluid or blood may be measured and obtained based on the potential difference formed between the reference comparison electrode and the working electrode. Thereby, the voltage generated by the working electrode can be obtained more accurately. In addition, in this case, the electronic apparatus 880 may automatically adjust and maintain the stability of the voltage at the working electrode according to a preset voltage value, so that the measured current signal can reflect the glucose concentration information in the tissue fluid or blood more accurately. In some examples, the number of the reference comparison electrodes can be one or plural, for example, two.

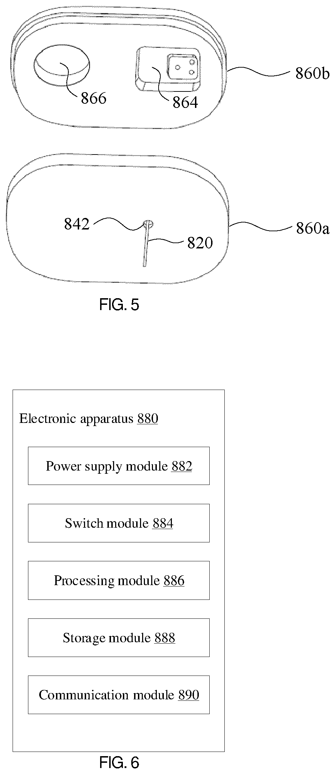

FIG. 4 is the schematic diagram of the first view angle of the application portion 860 according to an example of present embodiment. FIG. 5 is the schematic diagram of the second view angle of the application portion 860 according to an example of present embodiment.

In consideration of detaching, the application portion 860 may include a first upper body part 860a and a second upper body part 860b (see FIGs. 2-5). The first upper body part 860a and the second upper body part 860b may be detachably assembled and combined. The sensor 820 may be physically connected to the first upper body part 860a, and the sensor 820 may be electrically connected to the electronic apparatus 880 provided on the second upper body part 860 b.

In some examples, the sensor 820 can be assembled to the first upper body part 860a (see FIGs. 4-5) through the connecting seat 840. In addition, the connecting seat 840 can be integrally formed with the application portion 860. In addition, the connecting seat 840 can be provided on a side, close to the second upper body part 860b, of the first upper body part 860a (see FIG. 4).

To facilitate the sensor 820 to extend outward from an interior of the application portion 860, the connecting seat 840 may have a sensor coupling hole 842 (see FIG. 5). And, the sensor coupling hole 842 may extend through the first upper body part 860a (see FIG. 5). In some examples, an axis of the sensor 820 assembled to the connecting seat 840 may pass through the sensor coupling hole 842 (see FIG. 5). In this case, the coupling of a piercing member 260 (described later) with the sensor 820 through the sensor coupling hole 842 can be facilitated, thereby facilitating to place the sensor 820 at least partially subcutaneously on the host through the piercing member 260.

In consideration of electrical connection with the electronic apparatus 880, the connection part of the sensor 820 may have an electric contact 822 (see FIG. 4). The electric contact 822 may be electrically connected with the working electrode, the counter electrode, and the reference comparison electrode of the sensor 820, and in addition, the working electrode, the counter electrode, and the reference comparison electrode of the sensor 820 may be electrically connected with the electronic apparatus 880 through the electric contact 822. Thereby, it is facilitated for the glucose information acquired by the sensor 820 to be transmitted to the second upper body part 860b. The number of the electric contacts 822 can be 2, 3, or 4. The number of the electric contacts 822 may be the same as the number of the electrodes of the sensor 820.

In some examples, the connecting seat 840 may be of substantially columnar shape, for example, quadrangular shape. The examples of present embodiment are not limited hereof, in other embodiments, the connecting seat 840 may also be of substantially triangular shape, hexagonal shape, cylindrical shape, or hemispherical shape, etc.

In order to facilitate the incorporation of the first upper body part 860a and the second upper body part 860b, the second upper body part 860b may include a receiving portion 864 (see FIG. 5). The receiving portion 864 may be formed as a concave part matching the outer contour of the connecting seat 840, and the connecting seat 840 may be embedded in the receiving portion 864. In addition, the second upper body part 860b may further include a power supply chamber 866 (see FIG. 5). The power supply chamber 866 may be formed as a concave part used for mounting a power supply module, for example, such as a button battery. In addition, the second upper body part 860b may further include a removal button 868 (see FIG. 4) arranged opposite to the power supply chamber 866, and the button battery may be pushed out from the power supply chamber 866 by pressing the removal button 868. Thereby, the button battery may be easily replaced.

As described above, the electronic apparatus 880 of the medical instrument 800 may be provided at a second upper body part 860b. A contact of the electronic apparatus 880 for electrical connection to the outside may be arranged at the receiving portion 864 (see FIG. 5), as the first upper body part 860a is in assembling combination with the second upper body part 860b, the connecting seat 840 may be embedded in the receiving portion 864, and the electric contact 822 of the sensor 820 may be in contact with the contact of the electronic apparatus 880.

In the present embodiment, pushing the medical instrument 800 to the body surface of the host may include the following steps: applying the first upper body part 860a to the surface of the host and partially positioning the sensor 820 subcutaneously on the host; and assembling the second upper body part 860b to the first upper body part 860a. In this case, the second upper body part 860b without being placed subcutaneously can be reused.

In some examples, the application portion 860 is configured to adhere to the body surface of the host. For example, the medical instrument 800 may further include an adhesive sheet 870 provided on the first upper body part 860a and having adhesive properties (see FIG. 2). The first upper body part 860a may be adhered to the body surface of the host through the adhesive sheet 870. In some examples, an adhesive surface of the adhesive sheet 870 may be substantially equal to a bottom surface, close to the host, of the first upper body part 860a. In other examples, the adhesive surface of the adhesive sheet 870 may also be slightly larger than the bottom surface, close to the host, of the first upper body part 860a.

In some embodiments, the electronic apparatus 880 may receive the glucose information generated by the sensor 820. In some embodiments, the electronic apparatus 880 may further transmit the received glucose information to an external device, for example, a reading device 900 to be described as followed. In addition, as described above, the electronic apparatus 880 may further supply power to the sensor 820.

FIG. 6 is the schematic block diagram of the electronic apparatus 880 of the medical instrument 800 according to an example of the present embodiment.

In some examples, the electronic apparatus 880 may include a power supply module 882, a switch module 884 and a processing module 886 (see FIG. 6). The power supply module 882 may supply power to the sensor 820 and/or the processing module 886, the switch module 884 may control the connection on/off of the power supply module 882 and the sensor 820 and/or the processing module 886, and the processing module 886 may process signals (for example, glucose information) generated by the sensor 820.

In some examples, the electronic apparatus 880 may have an initial mode configured to be open and an operating mode configured to be closed. In this case, as in the initial mode, the electronic apparatus 880 is configured to be open, thereby power consumption can be saved before the electronic apparatus 880 enters the operating state. In some examples, the switch module 884 may be in an off state when the electronic apparatus 880 is configured to be in the initial mode, and the switch module 884 may be in a closed state when the electronic apparatus 880 is configured to be in the operating mode. That is, the electronic apparatus 880 may be switched from the initial mode to the operating mode by the switch module 884. In this case, the electronic apparatus 880 is controlled to be switched from the initial mode to the operating mode by the switch module 884, thereby effectively reducing the power consumption before operation.

In addition, the electronic apparatus 880 may further include a storage module 888 (see FIG. 6). The storage module 888 may store the signals generated by the sensor 820. In addition, the electronic apparatus 880 may further include a communication module 890 (see FIG. 6). The communication module 890 may communicate with an external apparatus (e.g., a smart terminal device). In some examples, the communication module 890 may be a Bluetooth module, an NFC module, a Wifi module, or the like. In some examples, the smart terminal device may be a smartphone, a smart watch, a tablet computer, a computer, or the like.

For instant transmission, the glucose information generated by the sensor 820 may be transmitted to the external apparatus after received by the electronic apparatus 880. Thereby, it is facilitated for the external apparatus to process or display the glucose information instantly. In addition, the electronic apparatus 880 may also be configured to store the glucose information generated by the sensor 820 in the storage module 888 after receiving the glucose information and then transmit the glucose information to the external apparatus at regular time.

As described above, the electronic apparatus 880 may be configured to communicate with the external equipment in a wireless communication mode or a wired communication mode. In this case, the electronic apparatus 880 is configured to communicate with the external equipment, so that analyte information acquired by the sensor 820 may be read in real time or at regular time. The external equipment may be a reading device 900.

As described above, the medical instrument 800 may be in communication connection with the reading device 900. In some examples, the reading device 900 may be a display having a communication function. In some examples, the communication function of the reading device 900 may be realized through a wireless communication mode or a wired communication mode. The wireless communication mode may be Bluetooth, NFC, Wifi or the like. The wired communication mode may be USB, optical fiber or the like.

In addition, the reading device 900 may also be an intelligent terminal installed with an application program matching the medical instrument 800. The intelligent terminal may be a notebook computer, a tablet computer, a smartphone or the like.

As described above, the medical instrument 800 according to the present embodiment may be applied to the host through the application apparatus 1000. Hereinafter, the application apparatus 1000 according to the embodiment is described in detail in conjunction with the accompanying drawings.

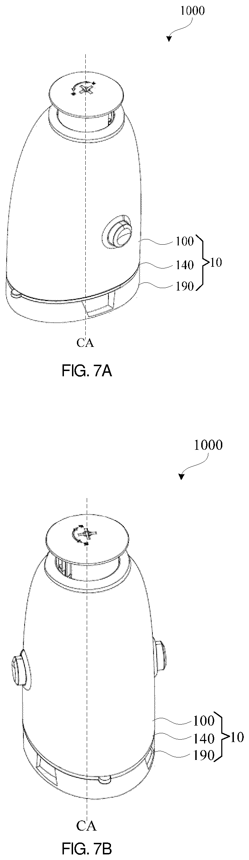

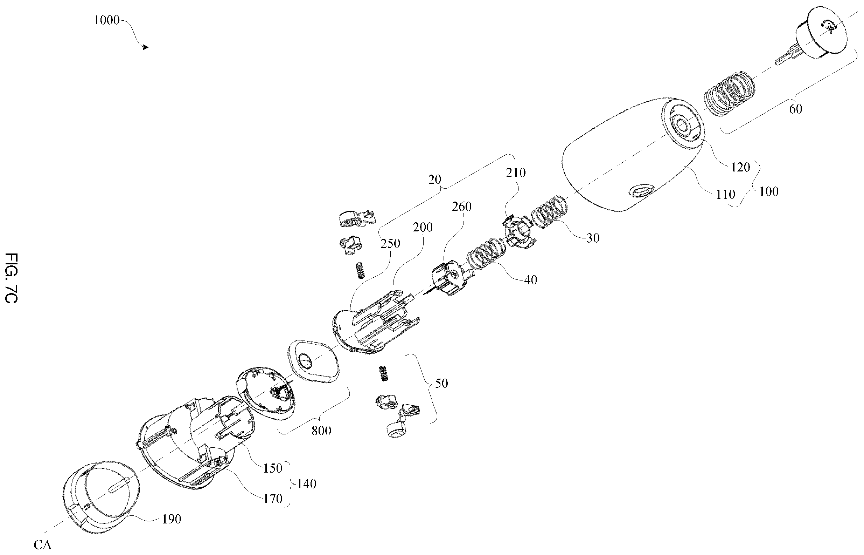

FIG. 7A is the overall appearance schematic diagram of the first view angle of the application apparatus 1000 according to an example of the present embodiment; FIG. 7B is the overall appearance schematic diagram of the second view angle of the application apparatus 1000 according to an example of the present embodiment; and FIG. 7C is a schematic explode diagram of the application apparatus 1000 according to an example of the present embodiment. In FIG. 7A, FIG. 7B and FIG. 7C, line CA schematically represents a central axis of the application apparatus 1000.

In the present embodiment, the application apparatus 1000 may include a housing 10 capable of providing a movement space, and an auxiliary mechanism 20 configured to move in the movement space of the housing 10 (see FIGs. 7A, 7B, and 7C). The housing 10 may have a proximate end close to the host in operation and a distal end far away from the host. The auxiliary mechanism 20 may be releasably held to the housing 10 and store the medical instrument 800. The auxiliary mechanism 20 may be configured to move relative to the housing 10 and be driven toward the host to apply the medical instrument 800 to the host when released. It is to be understood that, in the present disclosure, "proximate end" may be understood as an end that is close to the host in operation, and "distal end" may be understood as an end that is far away from the host in operation.

In some examples, the housing 10 may have the movement space, and the auxiliary mechanism 20 may be releasably held to the housing 10. In some examples, the auxiliary mechanism 20 may move in the movement space of the housing 10 when released. Additionally, in some examples, the housing 10 may also define an application position of the medical instrument 800 on the body surface of the host.

In some examples, the housing 10 may include a first housing 100 and a second housing 140. The movement space may be formed after the first housing 100 is assembled with the second housing 140. The first housing 100 may be releasably hold the auxiliary mechanism 20. The second housing 140 may define the application position of the medical instrument 800 on the body surface of the host and define a movement path of the auxiliary mechanism 20. The auxiliary mechanism 20 is capable of moving move relative to the second housing 140 along the movement path defined by the second housing 140 after released by the first housing 100 and applying the medical instrument 800 to the application position defined by the second housing 140. In the present embodiment, an axis of the first housing 100 and an axis of the second housing 140 may be substantially parallel to a central axis CA of the application apparatus 1000.

In some examples, the auxiliary mechanism 20 may include a moving main body 200, an accommodating portion 250, and the piercing member 260 (see FIG. 7C). The moving main body 200 may be releasably held in the first housing 100, and the moving main body 200 may be configured to move along the movement path defined by the second housing 140 when released. The accommodating portion 250 may be provided on the moving main body 200 and may be configured to be in combination with the medical instrument 800, the accommodating portion 250 may be driven toward the application position defined by the second housing 140. The piercing member 260 may be provided on the moving main body 200 in the manner of passing through the accommodating portion 250. The moving main body 200 may move toward the proximate end of the housing 10 after released, and the accommodating portion 250 and the piercing member 260 may also move toward the proximate end of the housing 10 so as to apply the medical instrument 800 stored within the accommodating portion 250 to the host. In some examples, the piercing member 260 may pierce subcutaneously the host so as to at least partially place the medical instrument 800 subcutaneously on the host.

In addition, in some examples, the application apparatus 1000 may further include a first driving mechanism 30 (see FIG. 7C). The first driving mechanism 30 may be configured to apply a force to the moving main body 200 in a manner of facing toward the proximate end. When released, the moving main body 200 may be driven by the first driving mechanism 30 towards the proximate end so as to push the medical instrument 800 stored the accommodating portion 250 towards the host, for example, to the application position defined by the second housing 140. In addition, the piercing member 260 may at least partially place the medical instrument 800 subcutaneously on the host.

In other examples, the application apparatus 1000 may also not include the first driving mechanism 30. In this case, when the moving main body 200 is released, the moving main body 200 may also be manually driven to move towards the proximate end.

In addition, in some examples, the piercing member 260 may be releasably provided on the moving main body 200, and the piercing member 260 may be configured to move relative to the accommodating portion 250 when released. For example, the piercing member 260 may move relative to the accommodating portion 250 in a direction far away from the host when released.

In addition, in some examples, the application apparatus 1000 may also include a second driving mechanism 40 (see FIG. 7C). The second driving mechanism 40 may be configured to apply a force to the piercing member 260 in a manner of facing toward the distal end. When the piercing member 260 is released, the piercing member 260 may be driven by the second driving mechanism 40 towards the distal end so as to move the piercing member 260 to be far away from the host.

In other examples, the application apparatus 1000 may also not include the second driving mechanism 40. In this case, the piercing member 260 may be manually driven away from the host. For example, in some examples, the piercing member 260 may also be integrally connected with the moving main body 200, the moving main body 200 may be manually moved in a direction far away from the host so as to move the piercing member 260 far away from the host.

In some examples, as described above, the housing 10 may include the first housing 100 (see FIG. 7A, FIG. 7B or FIG. 7C). In some examples, the first housing 100 may include a peripheral portion 110 and an end portion 120 (see FIG. 7C).

In some examples, the application apparatus 1000 may also include a disassembling mechanism 60 (see FIG. 7C). The disassembling mechanism 60 may be used to separate a sharp object of the piercing member 260 from a support seat (described later).

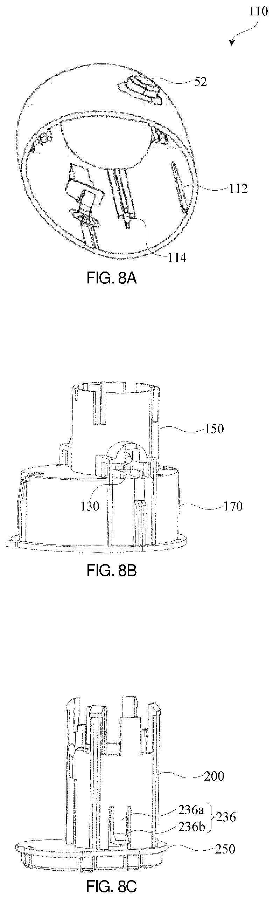

FIG. 8A is the perspective schematic diagram of the peripheral portion 110 of the first housing 100 according to an example of the present embodiment; FIG. 8B is the perspective schematic diagram of the second housing 140 according to this embodiment; and FIG. 8C is a perspective schematic diagram of the moving main body 20 according to an example of the present embodiment. In some examples, the peripheral portion 110 may be formed as a hollow cylindrical housing penetrating from top to bottom along the central axis CA of the application apparatus 1000 (see FIG. 8A), and the end portion 120 may be provided on the peripheral portion 110 in a manner of being close to the distal end of the housing 10. In some examples, the central axis CA may pass through a geometric center of the end portion 120.

In some examples, one or more rib-shaped protrusions may be provided on an inner wall of the first housing 100, and one or more rib-shaped grooves may be provided on an outer wall of the second housing 140. In some examples, when the second housing 140 is assembled to the first housing 100, the one or more rib-shaped protrusions of the first housing 100 may be respectively embedded into the one or more rib-shaped grooves of the second housing 140. In this case, the first housing 100 and the second housing 140 may be assembled conveniently by the matching of the rib-shaped protrusions and the rib-shaped grooves.

In some examples, peripheral ribs 112 extending substantially along the direction of the central axis CA may be provided on an inner wall of the peripheral portion 110 (see FIG. 8A). In some examples, the number of the peripheral ribs 112 may be plural, for example 2, 3, or 4. In some examples, the peripheral ribs 112 may serve as reinforcing ribs to effectively improve the deformation resistance of the peripheral portion 110. In some examples, the peripheral ribs 112 may also serve as guide ribs to facilitate the assembling of the first housing 100 and the second housing 140 (described later).

In some examples, connecting columns 114 which are substantially parallel to the central axis CA may be provided on the inner wall of the peripheral portion 110 (see FIG. 8A). In some examples, the number of connecting columns 114 may be plural, for example, 2, 3, or 4. In some examples, the end, close to the proximate end of the application apparatus 1000, of the connecting columns may be hot-type post end, i.e., the end is in a molten state by being heated and then may be solidified through cooling. In this case, the connecting columns 114 may cooperate with holes formed in the second housing 140 so as to be assembled in combination with the second housing 140 (described later).

In some examples, the second housing 140 may include a first holding portion 130 (see FIG. 8B). In some examples, the first holding portion 130 may be configured to releasably hold the auxiliary mechanism 20. In some examples, the first holding portion 130 may be provided on a sidewall 230 of the moving main body 200 (see FIG. 8B). In some examples, the first holding portion 130 may be an opening formed in the sidewall 230 of the moving main body 200.

In some examples, the interlocking between the first holding portion 130 and a held part (e.g. a first locking portion 236 of the moving main body 200 that will be described later) may be achieved by at least one of the structures of a buckle, a hook, a latch, or a pin. In this case, the first holding portion 130 and the first locking portion 236 are releasably interlocked by at least one of the structures of the buckle, the hook, the latch, or the pin, thereby providing an interlocking manner that is easy to release.

In some examples, the number of first holding portions 130 may be one or plural, such as 1, 2, 3 or 4. In the embodiment shown in the FIG. 8B, the number of the first holding portions 130 may be 2, and these two first holding portions 130 may be symmetrically disposed on the two opposite sides of the sidewall 230 of the moving main body 200.

In some examples, the first holding portion 130 may have a surface facing the distal end, and the first locking portion 236 may have a surface facing the proximate end, and when the moving main body 200 is held, the surface of the first holding portion 130 facing the distal end may be engaged with the surface of the first locking portion 236 facing the proximate end. In some examples, when the moving main body 200 is released, the surface of the first holding portion 130 facing the distal end may be separated from the surface of the first locking portion 236 facing the proximate end.

In some examples, the first locking portion 236 may include an arm 236a substantially extending along the direction of the central axis CA, and a protrusion 236b which is linked with the arm 236a and protrudes away from the central axis CA in the direction substantially orthogonal to the central axis CA (see FIG. 8C). In some examples, the arm 236a of the first locking portion 236 may be formed on the sidewall 230 of the moving main body 200. In this case, when the protrusion 236b of the first locking portion 236 is overlapped with the first holding portion 130, the protrusion 236b is actuated to move towards the central axis CA to be not overlapped with the first holding portion 130 any more, thereby it is facilitated for the first locking portion 236 to be released from the first holding portion 130.

Refer to FIG. 8A, FIG. 8B and FIG. 8C, a first triggering portion 50 is pressed towards the central axis CA, and the first triggering portion 50 is capable of actuating the first locking portion 236 to move towards the central axis CA, so that the overlapping relation with the first holding portion 130 may be released, and as a result, the first locking portion 236 may be released from the first holding portion 130.

In some examples, the arm 236a of the first locking portion 236 may be elastic. In some examples, the arm 236a of the first locking portion 236 may be elastic in a direction substantially orthogonal to the central axis CA. In some examples, the arm 236a of the first locking portion 236 is capable of expanding away from the central axis CA in a direction from the distal end to the proximate end. That is, by comparing the end, close to the proximate end, of the arm 236a and the end, close to the proximate end, of the arm 236a in a natural state (i.e., a state without external force), the end, close to the proximate end, of the arm 236a is farther from the central axis CA. In other examples, the arm 236a may be substantially parallel to the central axis CA. In some examples, when a force is applied to the arm 236a in a direction substantially orthogonal to the central axis CA, the arm 236a may be pivoted. For example, when the force is applied to the arm 236a in a direction substantially orthogonal to the central axis CA toward the central axis CA, the arm 236a may be pivoted so that the end, close to the proximate end, of the arm 236a moves toward the central axis CA. Thereby, the held member can be released with simple operation.

In some examples, the arm 236a of the first locking portion 236 is capable of expanding far away from the central axis CA in a direction from the distal end to the proximate end, and the protrusion 236b of the first locking portion 236 is capable of protruding in a direction away from the central axis CA. In this case, interlocking with the first holding portion 130 may be formed on the outside (away from the central axis CA) by expanding outwards the whole first locking portion 236, and the first locking portion 236 may be actuated toward the inside (close to the central axis CA), so that the first locking portion 236 can be released.

However, the examples of the present embodiment are not so limited hereof, The conception of the present invention is that the first holding portion 130 forms the structure such as the buckle, the hook, the latch, or the pin to hold the held member, and releases the held member by moving the buckle, the hook, the latch, or the pin away from the original position, based on this conception, the arm 236a of the first locking portion 236 and the protrusion 236b of the first locking portion 236 may also be closed up or protrude in other directions when buckling is formed.

In some examples, the protrusion 236b of the first locking portion 236 may have a surface facing the proximate end. In some examples, the surface, facing the proximate end, of the protrusion 236b may be substantially orthogonal to the central axis CA. The first locking portion 236 may be held and not moved toward to the proximate end by overlapping/abutting against the surface, facing the distal end, of the first holding portion 130. when the first locking portion 236 is actuated to move the surface, facing the proximate end, of the protrusion 236b away from the original position, that is, no longer abutting against the surface, facing the distal end, of the first holding portion 130, the first locking portion 236 may be released.

That is, the first locking portion 236 may be a structure such as the buckle, the hook, the latch, or the pin formed on a first limiting mechanism 150. For example, the first locking portion 236 may be formed as a structure which may be formed as a structure capable of being divided into at least two parts, such as a finger shape, a straight line shape, an "L" shape, a "J" shape, and a "Z" shape, at least one part (for example, the arm 236a connected to the sidewall 230 of the moving main body 200) may be pivoted, and the other part (for example, the protrusion 236b protruding outward from the arm 236a and abutting against the first holding portion 130) may move away from the initial position along with the pivoting, thereby releasing the hooking, hanging, latching, pushing, supporting, and the like of the held member.

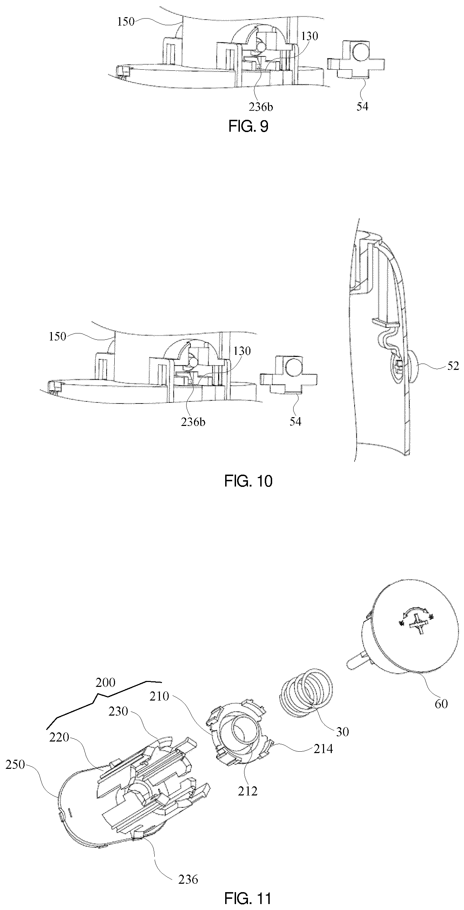

FIG. 9 is the partial schematic diagram of the first locking portion 236 and the first holding portion 130 when the moving main body 200 is held according to an example of the present embodiment. FIG. 10 is the partial schematic diagram of a first triggering mechanism 50 according to an example of the present embodiment.

A pressing part 52 of the first triggering mechanism 50 is pressed toward the central axis CA, the pressing part 52 is linked with a connecting piece 54, the connecting piece 54 abuts against the protrusion 236b and actuates the protrusion 236b toward the central axis CA, so that the protrusion 236b moves towards the central axis CA, the overlapping relation between the protrusion 236b and the first holding portion 130 is released, and as a result, the first locking portion 236 may be released from the first holding portion 130.

In some examples, as described above, the auxiliary mechanism 20 may include the moving main body 200, and the accommodating portion 250 provided on the moving main body 200. The moving main body 200 may be releasably held on the first housing 100. In some examples, the moving main body 200 may be releasably held by a first holding portion 130. The moving main body 200 may be driven toward the proximate end after released so as to push the medical instrument 800 accommodated in the accommodating portion 250 toward the host.

FIG. 11 is the perspective schematic diagram of the moving main body 200 according to an example of the present embodiment.

In some examples, a first driving portion 30 may be provided between the moving main body 200 and the first housing 100 (see FIG. 11). The first driving portion 30 may apply a force to the moving main body 200 in a direction toward the proximate end. The moving main body 200, when released, may be driven by the first driving portion 30 toward the proximate end so as to push the medical instrument 800 accommodated in the accommodating portion 250 toward the host.

In other examples, as described above, the application apparatus 1000 may also not include the first driving portion 30. In this case, the moving main body 200, when released, may also be manually pushed toward the host so as to push the medical instrument 800 accommodated in the accommodating portion 250 toward the host.

In some examples, the moving main body 200 may include a first bottom portion 210, a second bottom portion 220, and the sidewall 230 connecting the first bottom portion 210 and the second bottom portion 220. The first bottom portion 210 may be close to the distal end, the second bottom portion 220 may be close to the proximate end, and the accommodating portion 250 may be provided at the second bottom portion 220.

In some examples, a hollow portion may be formed between the first bottom portion 210, the second bottom portion 220, and the sidewall 230. In some examples, the piercing member 260 may be releasably provided within the hollow portion of the moving main body 200. In some examples, the piercing member 260 may move along the hollow portion of the moving main body 200 when released. In this case, the hollow portion is formed by the first bottom portion 210, the second bottom portion 220, and the sidewall 230, the accommodating portion 250 are provided at the second bottom portion 220, and the piercing member 260 is provided at the hollow portion, thereby facilitating movement of the piercing member 260 relative to the accommodating portion 250 when released. Thereby, when the medical instrument 800 is applied to the host, the piercing member 260 is released and moved relative to the accommodating portion 250, thereby enabling the piercing member 260 to be moved away from the host.

In other examples, the piercing member 260 may also be fixedly provided on the moving main body 200. In this case, when the medical instrument 800 is applied to the host, the moving main body 200 may be manually moved away from the host, thereby the piercing member 260 can also be moved away from the host.

In some examples, the first driving mechanism 30 may be provided between the first bottom portion 210 and the first housing 100. In some examples, a localization portion 212 used for positioning the first driving mechanism 30 may be provided on the first bottom portion 210. In this case, the position at which the first driving mechanism 30 applies force to the moving main body 200 may be more stably defined by the localization portion 212.

In some examples, the localization portion 212 of the first bottom portion 210 may be a columnar protrusion protruding toward the distal end along the central axis CA from the first bottom portion 210. In some examples, the localization portion 212 may be of cylindrical shape. In some examples, the localization portion 212 may be of solid cylindrical shape, and the first driving mechanism 30 may be provided on the periphery of the localization portion 212 in a sleeving manner. In other examples, the localization portion 212 may be of hollow cylindrical shape, and the first driving mechanism 30 may be provided on an outer periphery of the localization portion 212 in a sleeving manner and may also be embedded in an inner periphery of the localization portion 212.

In some examples, the first driving mechanism 30 may have an power storage state and an power release state, and when the first driving mechanism 30 is switched from the power storage state to the power release state, the first driving mechanism 30 may release power and apply a force to the moving main body 200 in a manner of facing the proximate end. When the moving main body 200 is held, the first driving mechanism 30 may exist between the moving main body 200 and the first housing 100 in the power storage state, and when the moving main body 200 is released, the first driving mechanism 30 may be switched from the power storage state to the power release state and apply the force to the moving main body 200 in the manner of facing the proximate end.

In some examples, the first driving mechanism 30 may have a compressed state and an extended state, and when the first driving mechanism 30 is switched from the compressed state to the extended state, the first driving mechanism 30 may release power. In some examples, the first driving mechanism 30 may be a compressible elastic member. In some examples, the first driving mechanism 30 may be a spring. In some examples, one end of the first driving mechanism 30 may be provided on the localization portion 212 of the first bottom portion 210 in a sleeving manner.

In some examples, the end portion 120 may have a localization portion. In some examples, the localization portion may be of cylindrical shape. In this case, the other end of the first driving mechanism 30 may be provided on the localization portion in a sleeving mode. In some examples, the localization portion may be of hollow cylindrical shape. In this case, the other end of the first driving mechanism 30 may be provided on the localization portion in a sleeving or embedding mode. In some examples, the localization portion may be composed of at least two sections of arc-shaped columns. In this case, the other end of the first driving mechanism 30 may be provided between the arc-shaped columns in an embedding mode.

In addition, the application apparatus 1000 of the embodiment is not limited hereof. In some examples, the application apparatus 1000 may not include the first driving mechanism 30. In this case, when the moving main body 200 is released, a force is manually applied to the moving main body 200 to move the moving main body 200 toward the proximate end.

In some examples, the moving main body 200 may include the first locking portion 236 configured to be releasably interlocked with the first holding portion 130. Thereby, the moving main body 200 may be releasably held on the first holding portion 130 through cooperation of the first locking portion 236 and the first holding portion 130.

In some examples, the first locking portion 236 may be provided on the sidewall 230 of the moving main body 200. That is, the first locking portion 236 may be provided on the sidewall 230 of the moving main body 200. The first locking portion 236 may be cooperated with the first holding portion 130 to enable the moving main body 200 to be held on the first housing 100.

In some examples, the number of first locking portions 236 may be one or plural, such as 1, 2, 3 or 4. In some examples, the number of the first locking portions 236 may be the same as the number of first holding portions 130.

In some examples, the first locking portion 236 may be formed as a buckling structure. Specifically, the first locking portion 236 may have the surface facing the proximate end, the first holding portion 130 may hold the moving main body 200 by overlapping, abutting or engaging on the surface, and the moving main body 200 may be released when the first holding portion 130 may move away from the surface. That is, the first holding portion 130 and the first locking portion 236 may be formed as an interlocking structure, projections of the first holding portion 130 and the first locking portion 236 have an overlapping part in a direction along the central axis CA, the first holding portion 130 may hold the first locking portion 236 through the overlapping part, and the first holding portion 130 and/or the first locking portion 236 may be moved away from each other to eliminate the overlapping part, so as to enable the first holding portion 130 to release the first locking portion 236. In some examples, the first holding portion 130 and/or the first locking portion 236 may be actuated in a mode of moving away from each other, thereby enabling the first locking portion 236 to be released by the first holding portion 130.

In addition, in some examples, the application apparatus 1000 may further include a first triggering mechanism 50 (see FIG. 7C) configured to separate the first holding portion 130 from the first locking portion 236 so as to release the moving main body 200. Thereby, it is facilitated for the first triggering mechanism 50 to release the moving main body 200.

In some examples, the application apparatus 1000 may further include the first triggering mechanism 50 (see FIG. 7C). The first triggering mechanism 50 is configured to trigger the first locking portion 236 so as to enable the moving main body 200 to be released by the first holding portion 130. In some examples, the first triggering mechanism 50 may be configured to actuate the first holding portion 130 and/or the first locking portion 236 in a mode of moving away from each other so as to release the moving main body 200. For example, the first triggering mechanism 50 may be configured to actuate the first locking portion 236 to move close to the central axis CA so as to release the first locking portion 236 from being held, thereby releasing the moving main body 200.

The first triggering mechanism 50 may include the pressing portion 52 provided on the first housing and a linking piece 54 linked with the pressing portion 52. A return member such as a spring may be provided between the linking member 54 and the sidewall 230.

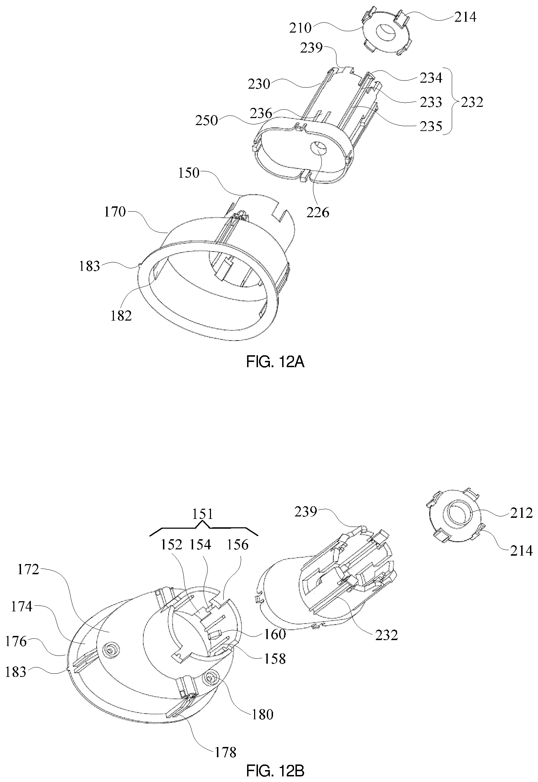

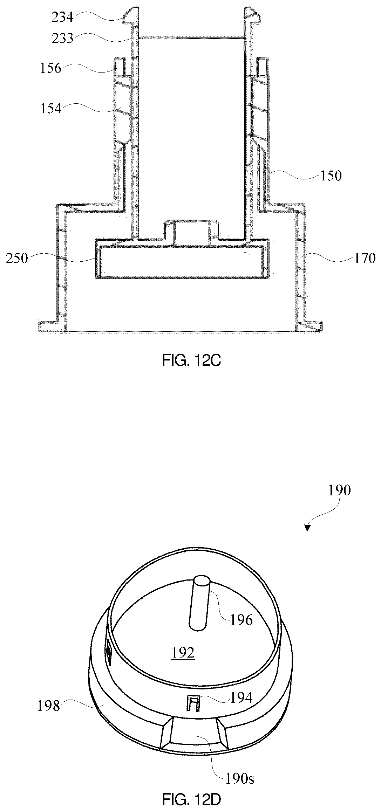

FIG. 12A is the schematic explode diagram of the first view angle of the second housing 140 and the auxiliary mechanism 20; FIG. 12B is the schematic explode diagram of the second view angle of the second housing 140 and the auxiliary mechanism 20; FIG. 12C is a cross-sectional schematic diagram of the auxiliary mechanism 20 assembled to the second housing 140; and FIG. 12D is a schematic diagram of a cover 190 according to this embodiment.

In some examples, the first bottom portion 210 of the moving main body 200 may be integrally formed with the sidewall 230. In other examples, the first bottom portion 210 of the moving main body 200 may be detachably assembled and coupled with the sidewall 230.

In some examples, the first bottom portion 210 may have a combination portion 214 (see FIG. 12A). In some examples, the number of combination portions 214 may be one or plural, such as 1, 2, 3, or 4. In some examples, the sidewall 230 may have a moving main body limitation application portion 238 (described later). The moving main body limitation application portion 238 may apply limitation to the piercing member 260 provided in the moving main body 200, thereby inhibiting undesired rotation of the piercing member 260. In some examples, the moving main body limitation application portion 238 may be formed as a groove and/or a ridge. In some examples, the moving main body limitation application portion 238 may be two ridge parts provided side-by-side and a groove between the two ridge parts. The combination portion 214 is coupled with the sidewall 230 by being engaged with ends of the two ridge parts of the moving main body limitation application portion 238.

In some examples, the second housing 140 may include a first limiting mechanism 150 and a second limiting mechanism 170. In some examples, the first limiting mechanism 150 and the second limiting mechanism 170 may communicate with each other. In some examples, the first limiting mechanism 150 and the second limiting mechanism 170 may be hollow cylindrical structures. In some examples, the first limiting mechanism 150 may releasably hold the moving main body 200 and limit a movement path of the moving main body 200. In some examples, the second limiting mechanism 170 may limit the application position of the medical instrument 800.

That is, in the application apparatus 1000 according to the present embodiment, the housing 10 may include the first housing 100 having the first holding portion 130, and the second housing 140 assembled to the first housing 100, the second housing 140 may have the first limiting mechanism 150 and the second limiting mechanism 170 that communicate with each other, the second limiting mechanism 170 may be configured to define the application position of the medical instrument 800, and the auxiliary mechanism 20 may be assembled to the first limiting mechanism 150 and may move along the first limiting mechanism 150 when the moving main body 200 is released by the first holding portion 130. In this case, the movement of the moving main body 200 is defined by the first limiting mechanism 150 of the second housing 140, and the application position of the medical instrument 800 is defined by the second limiting mechanism 170 of the second housing 140, thereby facilitating more accurate application of the medical instrument 800 to the host.

In some examples, the first limiting mechanism 150 may have a limitation application portion 151. The limitation application portion 151 may apply limitation to the moving main body 200, thereby suppressing the undesired rotation of the moving main body 200. In some examples, the limitation application portion 151 may be a groove and/or a ridge. In some examples, the limitation application portion 151 may include a ridge portion 154 which is provided on an inner wall of the first limiting mechanism 150 and extends substantially along the central axis CA. In some examples, the limitation application portion 151 may further include a groove 152 formed in the inner wall of the first limiting mechanism 150 and extending substantially along the central axis CA. In some examples, the length of the ridge portion 154 and the groove 152 may be smaller than the height of the first limiting mechanism 150, and the ridge portion 154 and the groove 152 may be substantially collinear along the direction of the central axis CA. In some examples, the limitation application portion 151 may further include a notch 156 formed in the first limiting mechanism 150, and the notch 156, the ridge portion 154, and the groove 152 may be sequentially formed from the proximate end to the distal end along the same straight line. In some examples, the number of application limiting parts 151 may be one or plural, such as 1, 2, 3 or 4. In some examples, a plurality of application limiting parts 151 may be uniformly distributed on the sidewall of the first limiting mechanism 150.

In some examples, the ridge portion 154 of the limitation application portion 151 may be cooperated with a limited portion 232 (described later) of the moving main body 200 including a groove structure, thereby suppressing undesired rotation of the moving main body 200. In some examples, the moving main body 200 may be assembled to the first limiting mechanism 150 along the groove 152, thereby the groove 152 may provide a guiding action at the time of assembling. In addition, in some examples, the notch 156 may be used for providing an engagement space for the protrusion 234 (described as follows) of the moving main body 200.

In addition, in some examples, the first limiting mechanism 150 may also include reinforcing ribs 158 provided on an inner wall (see FIG. 12B). In some examples, the reinforcing ribs 158 may extend substantially along the direction of the central axis CA. In some examples, the number of reinforcing ribs 158 may be plural, such as 2, 3, 4 and 6. In some examples, the reinforcing ribs 158 may be uniformly distributed on the inner wall of the first limiting mechanism 150.

In addition, in some examples, the first limiting mechanism 150 may further include a protrusion 160 (described in detail later) provided on an inner wall.

In addition, in some examples, the first limiting mechanism 150 may limit a stroke of the moving main body 200 in a direction along the central axis CA. In this case, the length of travel of the moving main body 200 in the direction along the central axis CA is limited by the first limiting mechanism 150, thereby enabling the medical instrument 800 accommodated in the accommodating portion 250 to be more accurately pushed to a predetermined distance, so that the medical instrument 800 can be more accurately applied to the host.

In addition, in some examples, projection is performed in the direction along the central axis CA, the accommodating portion 250 may be at least partially overlapped with the wall of the first limiting mechanism 150, and the end, close to the distal end, of the moving main body 200 may be at least partially overlapped with a wall of the first limiting mechanism 150 (see FIG. 12C). In this case, by enabling the accommodating portion 250 and the moving main body 200 to be at least partially overlapped with the wall of the first limiting mechanism 150, the length of travel of the moving main body 200 moving along the first limiting mechanism 150 may be effectively limited under a condition that the structure is simplified.

In addition, in some examples, a protrusion protruding away from the central axis CA in the direction substantially orthogonal to the central axis CA is provided at the end, close to the distal end, of the moving main body 200. In this case, the protrusion and the wall of the first limiting mechanism 150 may form buckling, and therefore excessive advancing of the moving main body 200 toward the proximate end may be effectively limited.

In addition, in some examples, the first limiting mechanism 150 may suppress the rotation of the moving main body 200. In this case, by suppressing the rotation of the moving main body 200, undesirable rotation of the piercing member 260 may be suppressed during application.

In addition, in some examples, the first limiting mechanism 150 may include a groove and/or a ridge which is continuously or discontinuously provided on the inner wall substantially along the direction of the central axis CA. In this case, by clamping the moving main body 200 with the groove and/or the ridge of the first limiting mechanism 150, undesirable rotation of the moving main body 200 within the first limiting mechanism 150 may be effectively suppressed.

In addition, in some examples, the moving main body 200 may include a groove and/or a ridge formed on an outer wall of the sidewall 230 substantially along the direction of the central axis CA. In this case, by clamping the groove and/or the ridge of the moving main body 200 with the groove and/or the ridge of the first limiting mechanism 150, the undesired rotation of the moving main body 200 within the first limiting mechanism 150 may be effectively suppressed.

In addition, in some examples, the first limiting mechanism 150 and the second limiting mechanism 170 may be of hollow cylindrical shape, and an inner diameter of the first limiting mechanism 150 may be not greater than the inner diameter of the second limiting mechanism 170. In this case, the first limiting mechanism 150 and the second limiting mechanism 170 are provided with hollow cylindrical shape, it is facilitated for the auxiliary mechanism 20 to move along the first limiting mechanism 150 and the second limiting mechanism 170.

In addition, in some examples, when the moving main body 200 is released, the moving main body 200 may move along the first limiting mechanism 150 and the accommodating portion 250 may move along the second limiting mechanism 170. In this case, the moving main body 200 moves along the first limiting mechanism 150 and the accommodating portion 250 moves along the second limiting mechanism 170, the medical instrument 800 accommodated in the accommodating portion 250 may be more accurately applied to a desired position of the body surface of the host.

In some examples, the limited portion 232 (see FIG. 12A and FIG. 12B) may be provided on the sidewall 230 of the moving main body 200. The limited portion 232 may be cooperated with the limitation application portion 151 of the first limiting mechanism 150 to suppress undesired rotation of the moving main body 200. In some examples, the limited portion 232 may include a groove 235 formed in an outer wall of the sidewall 230. In some examples, the groove 235 may be formed between two side-by-side ridges. In some examples, the limited portion 232 may further include an arm 233 formed by extending from the sidewall 230 to the distal end in a direction of the groove 235. In some examples, the limited portion 232 may further include the protrusion 234 protruding from the arm 233 away from the central axis CA in a direction substantially orthogonal to the central axis CA. In some examples, the number of limited portions 232 may be one or plural, such as 1, 2, 3 or 4. In some examples, the number of limited portions 232 may be the same as the number of limitation application portions 151. In some examples, a plurality of limited portions 232 may be uniformly distributed on the sidewall 230. In some examples, the arm 233 may be elastic.

In some examples, projection is performed in a direction substantially orthogonal to the central axis CA, a projection plane of the protrusion 234 of the limited portion 232 may be further away from the central axis CA than the projection of the notch 156 of the limitation application portion 151. In addition, the projection of the ridge 154 may be substantially engaged with the protrusion of the groove 235. In addition, the projection of the protrusion 234 may be generated engaged with the projection of the groove 152. In some examples, the length and/or width of the accommodating portion 250 may be greater than the diameter of the first limiting mechanism 150.

Hereinafter, refer to FIG. 12C, a force is applied to the moving main body 200 along a direction of a central axis CA, so that the moving main body 200 is assembled to the first limiting mechanism 150 through the second limiting mechanism 170. The protrusion 234 may slide into the groove 152, thereby providing a mechanism for assembling guidance. When the protrusion 234 abuts against the ridge 154, the ridge 154 extrudes the protrusion 234 and elastically deforms the arm 233 so that the protrusion 234 is moved toward the central axis CA and continues to slide across the ridge 154. When the protrusion 234 protrudes toward the distal end from the first limiting mechanism 150 along the direction of the central axis CA, the arm 233 returns to an initial state so that the protrusion 234 protrudes from the first limiting mechanism 150 substantially in a direction orthogonal to the central axis CA.

The moving main body 200 is assembled and coupled with the first limiting mechanism 150, and the groove 235 of the moving main body 200 may be clamped with the ridge portion 154 of the first limiting mechanism 150. Thereby, when the moving main body 200 moves along the first limiting mechanism 150, the moving main body 200 may be limited in the extending direction of the ridge portion 154. In addition, by clamping the groove 235 and the ridge portion 154, a mechanism for suppressing rotation may be provided, so that the undesired rotation of the moving main body 200 in the first limiting mechanism 150 may be limited.. In this case, when a piercing mechanism 260 is pierced subcutaneously to the host along with the moving main body 200, the undesired rotation is effectively suppressed, and as a result, discomfort of the host in the applying process may be reduced.

In addition, as described above, the projection is performed in a direction along the central axis CA, and the projection of the protrusion 234 is further away from the central axis CA than the projection of the notch 156. In this case, when the moving main body 200 is assembled and coupled with the first limiting mechanism 150, a buckling connection is formed by the protrusion 234 and the notch 156, thereby a displacement amount travelled by the moving main body 200 toward the proximate end may be limited.

In addition, as described above, in a direction substantially orthogonal to the central axis CA, the length and/or the width of the accommodating portion 250 connected to the second bottom portion 220 of the moving main body 200 is larger than the diameter of the first limiting mechanism 150. In this case, when the moving main body 200 is assembled and coupled with the first limiting mechanism 150, a displacement amount travelled by the moving main body 200 toward the distal end may be limited by the accommodating portion 250.

In some examples, the moving main body 200 may further include a limiting portion 239 formed on the sidewall 230, and the limiting portion 239 may have a protrusion toward the central axis CA. In this case, when the support seat of the piercing member is returned, the displacement of the limited portion 289 (described later) may be limited by the limiting portion 239.

In some examples, the second limiting mechanism 170 may include an upper bottom portion 172, a lower bottom portion 176, and a side portion 174 connecting the upper bottom portion 172 and the lower bottom portion 176 (see FIG. 12B). In some examples, the upper bottom portion 172 may be formed in such a manner that the end, close to the proximate end, of the first limiting mechanism 150 extends outward in a direction substantially orthogonal to the central axis CA. The side portion 174 may be of a hollow cylindrical structure, and an extending direction of the side portion 174 may be substantially parallel to the central axis CA. The lower bottom portion 176 may be formed in such a manner that the end, close to the proximate end, of the side portion 174 extends outward in a direction substantially orthogonal to the central axis CA.

In some examples, the width of the lower bottom portion 176 protruding from the side portion 174 in a direction substantially orthogonal to the central axis CA may be substantially equal to the thickness of the peripheral portion 110 of the first housing 100.

In some examples, the second limiting mechanism 170 may include rib grooves 178 (see FIG. 12B) provided on the side portion 174. In some examples, the number of rib grooves 178 may be one or plural, for example, 1, 2, 3, or 4. In some examples, the rib grooves 178 may be formed in such a manner that two ridges extending substantially along the direction of the central axis CA are provided side by side on the side portion 174, and the rib grooves 178 may be formed between the two side-by-side ridges.

In some examples, the rib grooves 178 may be cooperated with a peripheral portion rib 112 of the peripheral portion 110 to assemble and couple the first housing 100 with the second housing 140. In some examples, the number of rib grooves 178 may be the same as the number of peripheral portion ribs 112. In some examples, the peripheral portion rib 112 may be assembled into the rib groove 178 in a plug-in manner substantially along the direction of the central axis CA. In some examples, the inner contour of the peripheral portion 110 is substantially the same as the outer contour of the second limiting mechanism 170. When the first housing 100 and the second housing 140 are moved toward each other substantially along the direction of the central axis CA, the peripheral portion rib 112 may enter the rib groove 178 to assemble and couple the first housing 100 with the second housing 140.

In some examples, the second limiting mechanism 170 may further include assembly holes 180 (see FIG. 12B) provided on the upper bottom portion 172. In some examples, the number of the assembly holes 180 may be one or plural, such as 1, 2, 3 or 4. In some examples, the number of the assembly holes 180 may be the same as that of the connecting columns 114 of the first housing 100. The assembly holes 180 may be cooperated with the connecting columns 114 to enable the first housing 100 to be assembled and coupled with the second housing 140. The connecting columns 114 may be in butt joint with the assembly holes 180.

In some examples, the second limiting mechanism 170 may further include a flange 183 (see FIG. 12B). In some examples, the upper bottom portion 172 of the second limiting mechanism 170 may be further provided with a hollow structure. In this case, when the application apparatus 1000 and the box apparatus 2000 are assembled and coupled, internal and external air pressure is balanced by the hollow structure so as to facilitate the combination of the application apparatus 1000 and the box apparatus 2000m.

In some examples, the accommodating portion 250 may be moved along the second limiting mechanism 170. In some examples, when the application apparatus 1000 is operated, the second limiting mechanism 170 is applied to the body surface of the host, thereby limiting the application position of the medical instrument 800.

In addition, in some examples, side slots 182 are provided in an inner wall of the side portion 174 of the second limiting mechanism 170 (see FIG. 12A). In some examples, the number of side slots 182 may be one or plural, such as 1, 2, 3, or 4. In some examples, a plurality of side slots 182 may be uniformly distributed on the inner wall of the side portion 174 along a circumferential direction, or symmetrically distributed on the inner wall of the side portion 174. In some examples, the side slots 182 may extend substantially along a direction of the central axis CA. The side slots 182 may be cooperated with cover clamping portion 194 (described later) of the cover 190 so as to assemble and couple the second housing 140 with the cover 190.

In some examples, the housing 10 may further include the cover 190 which is detachably provided on the second housing 140 in a covering mode (see FIG. 7C).

In some examples, the cover 190 may include a cover bottom portion 192 and the cover clamping portion 194 provided on the cover bottom portion 192 (see FIG. 12D). The cover bottom portion 192 may be substantially of flat panel shape. The cover clamping portions 194 may be cooperated with the side slots 182 of the second housing 140 so as to assemble and couple the cover 190 with the second housing 140. The number of the cover clamping portions 194 may be one or plural, such as 1, 2, 3, or 4. The number of the cover clamping portion 194 may be equal to the number of the side slot 182. A plurality of cover clamping portions 194a and cover clamping portions 194b may be distributed on opposite sides of the cover bottom portion 192 along a symmetry axis of the cover 190.

In some examples, the cover clamping portion 194 may include an arm extending from the cover bottom portion 192 toward the distal end substantially along the central axis CA, and a protrusion protruding from an end portion of the arm in a direction away from the central axis CA. The arm of the cover clamping portion 194 may be elastic. When the cover 190 is assembled and coupled with the second housing 140, the protrusion of the cover clamping portion 194 may be clamped into the side slot 182 of the second housing 140.

In some examples, the cover 190 may further include a cover pillar 196 (see FIG. 12D). The axis of the cover pillar 196 may be substantially parallel to the central axis CA. In some examples, the cover pillar 196 may be formed by the cover bottom portion 192 extending toward the distal end along the central axis CA. In some examples, when the moving main body 200 is held, a vertical distance between the surface, facing the proximate end, of the medical instrument 800 coupled with the accommodating portion 250 and the lower bottom portion 176 of the second housing 140 may be substantially equal to the height of the cover pillar 196. In this case, when the cover 190 is assembled and coupled with the second housing 140, the medical instrument 800 coupled with the accommodating portion 250 is supported by the cover pillar 196, thereby the undesired release of the moving main body 200 due to, for example, a misoperation, may be further suppressed.

In some examples, the cover 190 may further include a flange 198 (see FIG. 12D). The position of the flange 198 of the cover 190 may correspond to the flange 183 of the second housing 140. Specifically, when the cover 190 is assembled and coupled with the second housing 140, projection is performed in a direction along the central axis CA, and a projection plane of the flange 198 may be substantially overlapped with a projection plane of the flange 183, or the projection plane of the flange 198 may substantially overlap the projection plane of the flange 183.

In some examples, the cover 190 may further include a step portion 199 which is formed by protruding from the cover bottom portion 192 along the central axis CA (see

FIG. 12D). When the cover 190 is coupled with the second housing 140, an upper surface of the step portion 199 (i.e., a surface close to the second housing 140) may abut against the lower bottom portion 176. In this case, the distance between the surface, facing the proximate end, of the medical instrument 800 coupled with the accommodating portion 250 and the lower bottom portion 176 of the second housing 140 along the direction of the central axis CA may be substantially equal to a height difference between a top end of the cover pillar 196 and the upper surface of the step portion 199.

In some examples, the upper surface of the step portion 199 may be partially concaved toward the cover bottom portion 192, thereby forming an operation space 190s (see FIG. 12D) for facilitating operation, for example, by hands. In other examples, the cover 190 may skip the step portion 199, alternatively, the cover bottom portion 192 may have a predetermined thickness, and the upper surface of the cover bottom portion 192 (i.e., the surface facing the second housing 140) may be partially concaved to form the operation space 190s. In this case, when the cover 190 is combined with the second housing 140, an operator may clamp the cover 190 through the operation space 190s, thereby facilitating operation to separate the cover 190 from the second housing 140.

FIG. 13A is the schematic explode diagram of the first view angle of the moving main body 200 and the piercing member 260 according to an example of the present embodiment; FIG. 13B is the schematic explode diagram of the second view angle of the moving main body 200 and the piercing member 260 according to an example of the present embodiment; FIG. 13C is the cross-sectional schematic diagram of the held piercing member 260 according to an example of the present embodiment; FIG. 13D is the cross-sectional schematic diagram of the released piercing member 260 according to an example of the present embodiment; and FIG. 13E is a cross-sectional schematic diagram of the moving main body 200 and the piercing member 260 in pre-assembling according to an example of the present embodiment.

In some examples, the moving main body 200 and the piercing member 260 may be integrally and fixedly connected. In other examples, the moving main body 200 and the piercing member 260 may be detachably assembled and coupled. In some examples, the piercing member 260 may be releasably held by the moving main body 200.

In some examples, the moving main body 200 may have the first bottom portion 210 close to the distal end, the second bottom portion 220 close to the proximate end, and the sidewall 230 connecting the first bottom portion 210 and the second bottom portion 220. In some examples, a hollow portion may be formed between the first bottom portion 210, the second bottom portion 220 and the sidewall 230. In some examples, the accommodating portion 250 may be provided on the second bottom portion 220 and face the proximate end. In some examples, the piercing member 260 may be provided in the hollow portion. In some examples, the second bottom portion 220 may have a through hole 226. In addition, in some examples, the piercing member 260 may pass through the through hole 226.

In some examples, the piercing member 260 may be releasably provided within the hollow portion. In addition, the piercing member 260 may be configured to move in the hollow portion relative to the moving main body 200 when released. In some examples, the second driving mechanism 40 may be provided between the second bottom portion 220 of the moving main body 200 and the piercing member 260. The second driving mechanism 40 may be configured to apply a force to the piercing member 260 toward the distal end. When the piercing member 260 is released, the second driving mechanism 40 may apply a force to the piercing member 260 toward the distal end so as to move the piercing member 260 away from the host. In some examples, the second driving mechanism 40 may be provided between the support seat 280 of the piercing member 260 and the second bottom portion 220 of the moving main body 200. Thereby, it is facilitated for the second driving mechanism 40 to apply a force to the piercing member 260 to be away from the accommodating portion 250.

In some examples, as described above, the moving main body 200 may include a sidewall 230 (see FIG. 13A and FIG. 13B). In some examples, the moving main body 200 may include the limitation application portion 238 provided on the sidewall 230. The piercing member 260 may have a limited portion 285 (described later). When the piercing member 260 is assembled and coupled with the moving main body 200, the moving main body 200 may apply limitation to the limited portion 285 through the limitation application portion 238 to limit the movement of the piercing member 260. In some examples, the number of limitation application portions 238 may be one or plural, such as 1, 2, 3, or 4. In some examples, a plurality of limitation application portions 238 may be uniformly distributed on the inner wall of the sidewall 230.

In some examples, the moving main body 200 may further include a second holding portion 242 (see FIG. 13A and FIG. 13B). The piercing member 260 may have a second locking portion 286. When the piercing member 260 is assembled and coupled with the moving main body 200, the moving main body 200 may be interlocked with the piercing member 260 by the second holding portion 242 to hold the piercing member 260. In some examples, the holding the second holding portion 242 of the piercing member 260 may be releasable. In some examples, the number of second holding portions 242 may be one or plural, such as 1, 2, 3, or 4. In some examples, a plurality of second holding portions 242 may be uniformly distributed on the sidewall 230.

In addition, in some examples, the moving main body 200 may be configured to suppress the rotation of the piercing member 260. In this case, by suppressing undesired rotation of the piercing member 260 during application, the user experience during application of the medical instrument 800 may be improved. In some examples, the moving main body 200 may suppress undesired rotation of the piercing member 260 during application through the limitation application portion 238.

In addition, in some examples, the moving main body 200 may include the groove and/or the ridge which is provided on the inner wall of the sidewall 230 substantially along the direction of central axis CA. In this case, by cooperation of the groove and/or the ridge provided on the inner wall of the sidewall of the moving main body 200 with the groove and/or the ridge of the piercing member 260, the undesired rotation of the piercing member 260 during application may be effectively suppressed. That is, in some examples, the limitation application portion 238 of the moving main body 200 may be the groove and/or the ridge provided on the inner wall of the sidewall 230 substantially along the direction of the central axis CA.

In some examples, the limitation application portion 238 of the moving main body 200 may be formed as a groove structure (see FIG. 13A and FIG. 13B). In some examples, the limitation application portion 238 may include two ridges provided side by side on the inner wall of the sidewall 230 substantially along the direction of the central axis CA, and a groove formed between the two ridges.

In addition, in some examples, the moving main body 200 may have a notch 242 substantially along the direction of the central axis CA. In this case, by providing the notch 242 in the moving main body 200 along the direction of the central axis CA, it is facilitated to apply a force to the piercing member 260 provided in the hollow portion of the moving main body 200.

In addition, in some examples, the protrusion 160 protruding toward the central axis CA through the notch 242 may be provided on the inner wall of the first limiting mechanism 150. In this case, when the moving main body 200 moves along the first limiting mechanism 150, the protrusion 160 provided on the inner wall of the first limiting mechanism 150 can apply a force on the piercing member 260 through the notch 242 of the moving main body 200, and thereby a triggering mechanism for the piercing member 260 may be provided in case of simplified structure.

In some examples, the second holding portion 242 of the moving main body 200 may be a notch formed on the sidewall 230 (see FIG. 13A and FIG. 13B). In some examples, the second holding portion 242 may extend substantially along the central axis CA and may have a first notch 244 and a second notch 246 which are communicated with each other. The first notch 244 may be close to the proximate end, and the second notch 246 may be close to the distal end. In some examples, the width of the first notch 244 may be different from that of the second notch 246. In some examples, the width of the first notch 244 may be larger than that of the second notch 246.

In some examples, the accommodating portion 250 may have a restraining portion 252 used for restraining the application portion 860. In this case, the application portion 860 of the medical instrument 800 accommodated in the accommodating portion 250 is restrained by the restraining portion 252, thereby accommodating the medical instrument 800 more stably.

In some examples, the restraining portion 252 of the accommodating portion 250 may include an arm 254 and a protruding portion 256. The arm 254 may extend outward from a bottom portion of the accommodating portion 250 substantially in the same direction as the side portion. In some examples, the arm 254 may be positioned substantially in the same circumferential direction as the side portion of the accommodating portion 250. In some examples, the arm 254 may be elastic. In some examples, the arm 254 may draw close to the central axis CA. In some examples, the protruding portion 256 may be a protrusion provided on an end of the arm 254. In some examples, the protruding portion 256 may protrude toward the central axis CA. In this case, the restraining portion 252 is capable of restraining the first upper body part 860a with overall size smaller than the accommodating portion 250, for example, the first upper body part 860a may be provided inside the accommodating portion 250 in an embedding mode, and the restraining portion 252 may be in interference fit with an outer wall of the first upper body part 860a.

In other examples, the protruding portion 256 may also protrude away from the central axis CA. In this case, the restraining portion 252 is capable of restraining the first upper body part 860a with the overall size larger than that of the accommodating portion 250. For example, the first upper body part 860a may be provided on the outer side of the accommodating portion 250 in a sleeving mode, and the restraining portion 252 is in interference fit with the inner wall of the first upper body part 860a.

In some examples, the number of the restraining portion 252 may be one or plural, for example, 1, 2 3 or 4. In some examples, the restraining portion 252 may be uniformly distributed along the side portion of the accommodating portion 250.

In some examples, the piercing member 260 may include a sharp object 270, and the support seat 280 (see FIG. 13A and FIG. 13B) for supporting the sharp object 270. In some examples, the support seat 280 of the piercing member 260 may be provided in the hollow portion of the moving main body 200, the accommodating portion 250 may be provided on the second bottom portion 220, the second bottom portion 220 may have the through hole 226, and the sharp object 270 may pass through the through hole 226 of the second bottom portion 220. In this case, the sharp object 270 may be engaged with the medical instrument 800 accommodated in the accommodating portion 250 by passing through the through hole 226 of the second bottom portion 220, thereby it is facilitated for the medical instrument 800 to be at least partially placed subcutaneously on the host through the piercing member 260.

In addition, in some examples, the moving main body 200 may further include a structure 224 (see FIG. 13C) provided on the second bottom portion 220 and used for positioning the second driving mechanism 40. In some examples, the structure 224 may be of cylindrical shape. In some examples, the structure 224 may be of hollow cylindrical shape. In this case, the second driving mechanism 40 may be localized by being provided on the structure 224 in sleeving manner, thereby enabling to limit a driving position of the second driving mechanism 40.

In addition, in some examples, when the piercing member 260 is held, a distance between the support seat 280 and the first bottom portion 210 may be not less than the length of the sharp object 270 protruding from the accommodating portion 250. In this case, by setting the distance between the support seat 280 and the first bottom portion 210 to be not less than the length of the sharp 270 protruding from the accommodating portion 250, a movement space can be provided for the piercing member 260 to leave the host, thereby reducing undesired damage of the piercing member 260 to the host.

In some examples, the sharp object 270 and the support seat 280 may be integrally formed. In other examples, the sharp object 270 and the support seat 280 may be configured to be detachably assembled and combined. Thereby, it is facilitated for the sharp object 270 to be sterilized independently.

In some examples, the limited portion 285 of the piercing member 260 may be formed as a ridge structure (see FIG. 13B). In some examples, the limited portion 285 may include two grooves formed in the sidewall of the support seat 280 in a side-by-side mode substantially along the direction of the central axis CA, and a ridge formed between the two grooves. In some examples, the number of the limited portions 285 may be one or plural, such as 1, 2, 3 or 4. In some examples, the number of the limited portions 285 may be equal to the number of limitation application portions 238.

In addition, in some examples, the support seat 280 may include a groove and/or a ridge provided on an outer wall substantially along the direction of the central axis CA. In this case, by cooperation of the groove and/or the ridge of the support seat 280 with the groove and/or the ridge on the inner wall of the sidewall of the moving main body 200, the undesired rotation of the piercing member 260 may be effectively suppressed. That is, the limited portion 285 of the piercing member 260 may be formed as a groove and/or a ridge provided on the outer wall substantially along the direction of the central axis CA. The limited portion 285 of the piercing member 260 may be cooperated with the limitation application portion 238 of the moving main body 200 to suppress the undesired rotation of the piercing member 260.

In addition, in some examples, the piercing member 260 may include the second locking portion 286 configured to releasably interlock with the second holding portion 242. Thereby, the piercing member 260 may be releasably held to the second holding portion 242 by cooperation of the second locking portion 286 with the second holding portion 242. In some examples, the second locking portion 286 may be provided on the support seat 280 of the piercing member 260.

In some examples, the second locking portion 286 may include an arm 287 extending substantially along the direction of the central axis CA, and a protrusion 288 interlocked with the arm 287 and protruding substantially in a direction orthogonal to the central axis CA away from the central axis CA. In this case, when the piercing member 260 is placed in the hollow portion of the moving main body 200, the piercing member 260 may be held with a simplified structure by the second locking portion 286 overlapping with the notch 242 of the moving main body 200. In some examples, the arm 287 of the second locking portion 286 may be elastic in a direction substantially orthogonal to the central axis CA of the application apparatus 1000.

That is, in some examples, the moving main body 200 may have the notch 242 substantially along the direction of the central axis CA. In some examples, the protrusion 288 of the second locking portion 286 may pass through the notch 242 when the piercing member 260 is held. In this case, the second locking portion 286 may be held with a simplified structure by overlapping the protrusion 288 of the second locking portion 286 with the notch 242 of the moving main body 200.

In addition, in some examples, the arm 288 of the second locking portion 286 may be extruded toward the central axis CA when the piercing member 260 is released. Thereby, the second locking portion 286 may be released with simple operation.

In addition, in some examples, the second holding portion 242 may be releasably interlocked with the second locking portion 286 by at least one structure of the buckle, the hook, the latch, or the pin. In this case, the second holding portion 242 may be releasably interlocked with the second locking portion 286 by at least one structure of the buckle, the hook, the latch, or the pin, thereby providing an interlocking manner facilitating to be released.

In some examples, the second locking portion 286 of the piercing member 260 may be formed as a shoulder structure (see FIG. 13A and FIG. 13B). In some examples, the second locking portion 286 may include the arm 287 extending toward the distal end substantially along the direction of the central axis CA, and the protrusion 288 protruding from an end of the arm 287 in a direction substantially orthogonal to and away from the central axis CA. In some examples, the arm 287 may be elastic. In some examples, the arm 287 may be gradually away from the central axis CA along the direction of the central axis CA and toward the distal end. In some examples, a projection plane of the protrusion 288 may be entirely or partially overlapped with a projection plane of the first notch 244 when projecting along the direction of the central axis CA. In some examples, the number of the second locking portions 286 may be one or plural, such as 1, 2, 3, or 4. In some examples, the number of the second locking portions 286 may be equal to the number of second holding portions 242.

In addition, in some examples, the width of the surface, facing the distal end of the protrusion 288 may be smaller than the width of the first notch 244 and may be greater than the width of the second notch 246.

In some examples, when the first limiting mechanism 150 and the moving main body 200 are assembled, the protrusion 160 provided on the inner wall of the first limiting mechanism 150 may pass through the second notch 246 and the first notch 244 in sequence. In this case, by providing the first notch 244, a holding mechanism for the piercing member 260 may be provided, and in addition, by providing the second notch 246, a structure facilitating assembling may be provided. The protrusion 160 passes through the second notch 246 and enters the first notch 244, and the protrusion 288 of the second locking portion 286 protrudes through the first notch 244, thereby providing a holding mechanism for the piercing member 260, and in addition, the protrusion 160 may pass through the first notch 244 to abut against the protrusion 288 of the second locking portion 286, thereby providing a triggering mechanism that triggers the second locking portion 286 to be released through the protrusion 160 and the first notch 244.

As shown in FIG. 13E, a force is applied to the piercing member 260 toward the proximate end to enable the piercing member 260 to enter the hollow portion of the moving main body 200, and the limited portion 285 of the piercing member 260 is aligned one by one with the limitation application portion 238 of the moving main body 200, and the limited portion 285 may be clamped and embedded in the limitation application portion 238 (shown in the FIG. 13E). Thereby, on one hand, a guide is provided for the assembling of the piercing member 260 and the moving main body 200; and on the other hand, the piercing member 260 assembled in the moving main body 200 is limited by the limitation application portion 238, and the undesired rotation may be effectively suppressed.

In addition, as shown in FIG. 13C and FIG. 13D, when the protrusion 288 of the second locking portion 286 of the piercing member 260 is moved to the first notch 244, due to no extrusion of the inner wall of the sidewall 230, the arm 287 returns to the initial state, that is, the projection plane of the protrusion 288 is completely or partially overlapped with the projection plane of the first notch 244 along the central axis CA; and since the width of the surface, facing the distal end, of the protrusion 288 is larger than the width of the second notch 246, the protrusion 288 is placed in the first notch 244 and the surface, facing the distal end, of the protrusion 288 abuts against a junction of the first notch 244 and the second notch 246 (see FIG. 13C), thereby being held in the hollow portion of the moving main body 200. When a force is applied to the arm 287 to pivot toward the direction of the central axis CA, the protrusion 288 is moved toward the direction of the central axis CA, that is, gathered together inward. In this case, the surface, facing the distal end, of the protrusion 288 no longer abuts against the junction of the first notch 244 and the second notch 246, thereby enabling the piercing member 260 to be released by the moving main body 200 (see FIG. 13D).

In some examples, when the piercing member 260 is released by the moving main body 200, the second driving mechanism 40 drives the piercing member 260 toward the distal end to enable the piercing member 260 to leave the host.

In some examples, the second driving mechanism 40 may have the same or similar configuration as the first driving mechanism 30, as for the features of the second driving mechanism 40, refer to the features description of the first driving mechanism 30. In some examples, the second driving mechanism 40 may be a spring. Thereby, an actuation mechanism may be provided for the piercing member 260 with a simplified structural design.

In some examples, the second driving mechanism 40 may be provided between the support seat 280 and the second bottom portion 220, and when the piercing member 260 is held, the distance between the support seat 280 and the first bottom portion 210 is not less than the depth of subcutaneous implantation. In this case, by providing sufficient movement space to the piercing member 260, it is facilitated for the piercing member 260 to leave the host.

In some examples, the application apparatus 1000 further provides a triggering mechanism through which the interlocking to the second locking portion 268 may be released by the second holding portion 242, and thus the piercing member 260 may be released from the moving main body 200. That is, in some examples, the application apparatus 1000 further includes a second triggering mechanism configured to release the second holding portion 242 from the second locking portion 286 so as to release the piercing member 260. Thereby, it is facilitated for the piercing member 260 to be released through the second triggering mechanism.

As described above, the second locking portion 286 is formed as a shoulder structure that may abut against the second holding portion 242, thereby being held by the second holding portion 242 in the form of buckling. In some examples, such a triggering mechanism is provided to actuate the second holding portion 242 or the second locking portion 286 to separate the second holding portion 242 from the second locking portion 286.

In some examples, the triggering mechanism acting on the second holding portion 242 and the second locking portion 286 may be provided on an advancing path of the second locking portion 286. The triggering mechanism may be employed when the moving main body 200 is moved toward the proximate end. In some examples, the triggering mechanism may be a protruding portion provided on the advancing path of the second locking portion 286. In this case, when the second locking portion 286 passes the protruding portion, the arm 287 of the second locking portion 286 may be extruded toward the central axis CA and elastically deformed to separate a protrusion 288 of the second locking portion 286 from the first notch 244 and the second notch 246.

In some examples, as described above, the protrusion 160 is provided on the inner wall of the first limiting mechanism 150 (see FIG. 12B). When the moving main body 200 moves along the first limiting mechanism 150, the first notch 244 and the second notch 246 of the moving main body 200 may pass through the protrusion. In addition, when the piercing member 260 is held in the hollow portion of the moving main body 200, the second locking portion 286 of the piercing member 260 is deformed due to being extruded by the protrusion 160 and leaves the junction of the first notch 244 and the second notch 246, thereby enabling the piercing member 260 to be released.

In addition, when the moving main body 200 and the first limiting mechanism 150 are assembled, the protrusion 160 may pass through the second notch 246 and the first notch 244 in sequence until the moving main body 200 and the first limiting mechanism 150 are assembled and coupled. By the protrusion 160 provided on the inner wall of the first limiting mechanism 150 and the first notch 244 and the second notch 246 provided on the sidewall 230 of the moving main body 200, an advancing triggering mechanism may be provided without adversely affecting the assembling of the moving main body 200 and the first limiting mechanism 150.

Now, the application process of the application apparatus 1000 is re-described: the application of the medical instrument 800 to the host by the application apparatus 1000 may include an application phase, and a withdrawal phase. In the application phase, the medical instrument 800 may be applied to the host; and in the withdrawal phase, the piercing member 260 may be removed from the host.

In the application phase, the pressing portion 502 of the first triggering mechanism 50 is pressed to move an actuating portion 504 of the first triggering mechanism 50 toward the proximate end; then, when the actuating portion 504 moves to the first holding mechanism 130, the actuating portion 504 actuates the first holding mechanism 130 to pivot in a direction away from the central axis CA (see FIG. 10I and FIG. 10J) so as to separate the first holding portion 130 from the first locking portion 236 (see FIG. 9B and FIG. 9C), thereby enabling the moving main body 200 to be released; then, the first driving mechanism 30 applies a force to the moving main body 200 in a manner of facing the proximate end so as to move the moving main body 200 toward the proximate end; then, under the action of the piercing member 260, the first upper body part 860a combined with the accommodating portion 250 is pushed to the surface of the host and the sensor 820 is at least partially placed subcutaneously on the host. Then, the second upper body part 860b is combined with the first upper body part 860a.

In some examples, the application apparatus 1000 may also not include the first driving mechanism 30. In this case, when the moving main body 200 is released, the moving main body 200 may also be manually driven toward the host, thereby applying the medical instrument 800 accommodated in the accommodating portion 250 to the host through the piercing member 260.

In the withdrawal phase, as the moving main body 200 moves toward the proximate end along the first limiting mechanism 150, the protrusion 160 provided on the inner wall of the first limiting mechanism 150 and the second locking portion 286 of the piercing member 260 are close to each other through the first notch 244 provided on the moving main body 200, and the protrusion 160 extrude the second locking portion 286 to pivot the second locking portion 286 in a direction close to the central axis CA, so that the second locking portion 286 is separated from the second holding portion 242 (see FIG. 13C and FIG. 13D); and then, the second driving mechanism 40 applies a force to the piercing member 260 to move toward the distal end, so that the piercing member 260 moves toward the distal end and leaves the host.

In some examples, the piercing member 260 may also be fixedly connected with the moving main body 200. In addition, in some examples, the application apparatus 1000 may also not include the second driving mechanism 40. In this case, after the medical instrument 800 is applied to the host, the whole moving main body 200 may be manually driven in the direction away from the host, and thereby the piercing member 260 fixedly connected with the moving main body 200 may leave the host along with the moving main body 200.

In addition, it needs to be noted that in the withdrawal phase, the first driving mechanism 30 (or manual operation) may still apply a force to the moving main body 200 toward the host. In other words, for the first driving mechanism 30 configured as an elastic component (e.g., a spring), when the moving main body 200 is driven toward the host and the medical instrument 800 accommodated in the accommodating portion 250 is applied to the body surface of the host, the first driving mechanism 30 may still be in an power storage state. In this case, in the withdrawal phase, the medical instrument 800 is tightly attached to the body surface of the host under the action of the first driving mechanism 30, and thereby the possibility that the medical instrument 800 is separated from the host in the return phase can be effectively reduced.

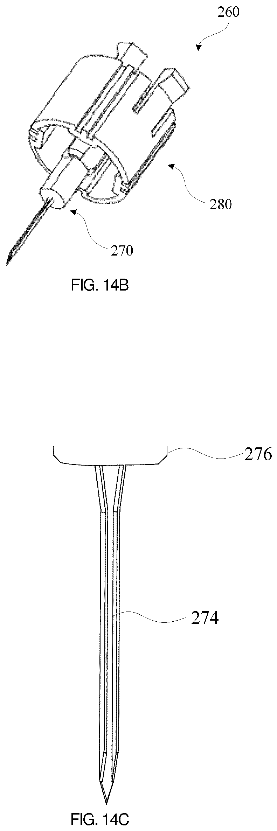

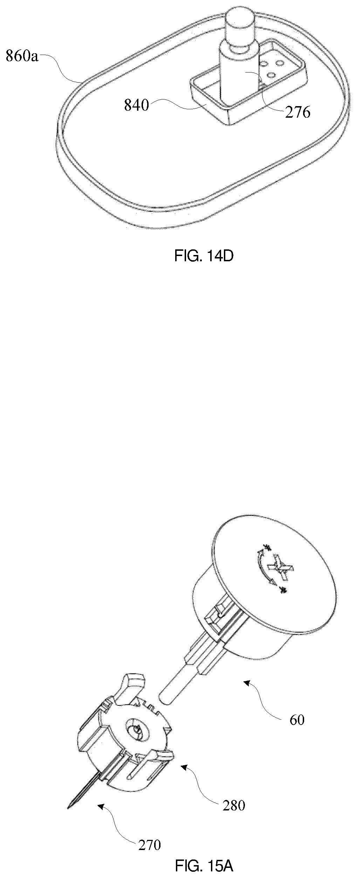

FIG. 14A is the schematic explode diagram of the piercing member 260 according to an example of the present embodiment; FIG. 14B is the assembly schematic diagram of the piercing member 260 according to an example of the present embodiment; FIG. 14C is the enlargement schematic diagram of the needle-shaped portion 272 according to an example of the present embodiment; and FIG. 14D is the assembly schematic diagram of the sharp object 270 and the sensor 820 according to an example of the present embodiment.

As described above, in the present embodiment, the piercing member 260 may include the sharp object 270, and the support seat 280 for supporting the sharp object 270 (see FIG. 14A). In some examples, the sharp object 270 may be integrally formed with the support seat 280. In other examples, the sharp object 270 may be detachably assembled with the support seat 280. In some examples, the sharp object 270 may have a groove, and the medical instrument 800 may be fully or partially provided in the groove. In some examples, the second driving mechanism 40 may apply a force to the support seat 280. In this case, by fully or partially placing the medical instrument 800 in the groove of the sharp object 270, it is facilitated for the medical instrument 800 to be at least partially placed subcutaneously on the host through the piercing member 260. In some examples, an implanting part of the sensor 820 may be arranged in the groove 274 of the sharp object 270.

In some examples, the sharp object 270 may include the needle-shaped portion 272 (see FIG. 14A). In some examples, the groove 274 extending along the length direction of the needle-shaped portion 272 may be formed on the needle-shaped portion 272. The medical instrument 800 may be at least partially provided in the groove 274 of the sharp object 270. In this case, the sensor 820 may be received in the groove 274 so as to be placed subcutaneously on the host along with the sharp object 270.

In some examples, the sharp object 270 may include a cap portion 276 connected to the needle-shaped portion 272 (see FIG. 14A). In some examples, the needle-shaped portion 272 may be assembled to the support seat 280 through the cap portion 276. In some examples, the cap portion 276 may include a first part 277, a second part 278, and a third part 279 which are connected in sequence. In some examples, the first part 277, the second part 278, and the third part 279 may be three columnar structures with different diameters. In addition, in some examples, the diameter of the second part 278 may be smaller than the diameter of the first part 277 and smaller than the diameter of the third part 279, thereby enabling to form an annular concave portion.

In some examples, the support seat 280 may include sharp object fixing portion 282 (see FIG. 14A). In some examples, the number of the sharp object fixing portion 282 may be plural, such as 2, 3, or 4. In addition, a plurality of sharp object fixing portions 282 may be circumferentially provided about the central axis CA so as to clamp the cap portion 276. In some examples, the support seat 280 may have a plurality of finger-shaped portions that are gathered together, and the cap portion 276 may be releasably clamped by the plurality of finger-shaped portions. That is, the sharp object fixing portions 282 of the support seat 280 may be formed as the plurality of finger-shaped portions that are gathered together. In this case, the cap portion 276 of the sharp object 270 is cooperated with the plurality of finger-shaped parts of the support seat 280, thereby facilitating assembling and disassembling between the sharp object 270 and the support seat 280.

In some examples, the sharp object fixing portion 282 may gradually gather towards the central axis CA along the direction of the central axis CA and toward the proximate end. Thereby, a mechanism for clamping the cap portion 276 may be formed. In some examples, the sharp object fixing portion 282 may include an arm 283 extending substantially along the direction of the central axis CA, and a protrusion 284 outwards protruding from the end portion of the arm 283 to the central axis CA (see FIG. 14A). In some examples, the arm 283 may be elastic. When the sharp object 270 is assembled and coupled with the support seat 280, the protrusion 284 of the sharp object fixing portion 282 may be embedded into the annular concave portion of the cap portion 276. The first part 277, the third part 279 and the annular concave portion are cooperated with one another, so that the sharp object 270 is fixed. The first part 277 and the third part 279 respectively abutt against the protrusion 284 from two directions. Thereby, the undesired movement of the sharp object 270 relative to the support seat 280 along the central axis CA may be suppressed.

In some examples, the sharp object 270 of the piercing member 260 may be assembled with the sensor 820. Specifically, as shown in FIG. 14D, in some examples, the needle-shaped portion 272 of the sharp object 270 may be placed in the sensor coupling hole 842 in the connecting portion 840, and the implanting part and an extending part of the sensor 820 may be placed in the groove 274 of the sharp object 270.

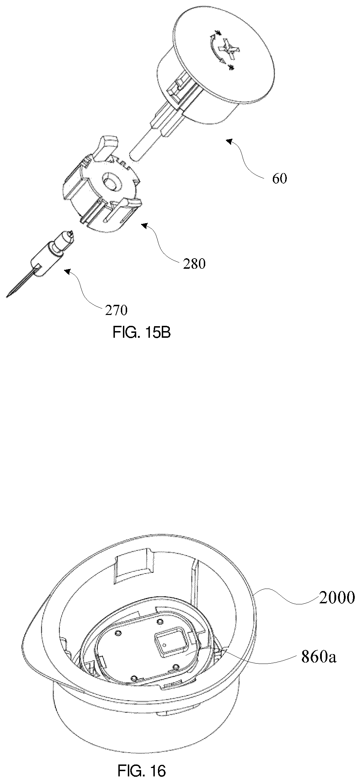

FIG. 15A is the schematic diagram of the sharp object 270 without being disassembled; and FIG. 15B is a schematic diagram of the sharp object 270 disassembled. In some examples, the disassembling mechanism 60 may have an elongated member.

In some examples, the piercing member 260 may have the through hole 281 penetrating from a distal end surface to a proximate end surface, and the through hole 281 may communicate with the sharp object fixing portion 282. The elongated member of the detaching mechanism 60 may push the sharp object 270 through the through hole 281, thereby disassembling the sharp object 270 from the sharp object fixing portion 282.

FIG. 16 is the perspective schematic diagram of the box apparatus 2000 according to the present embodiment. In the present embodiment, the first upper body part 860a, the sensor 820 and the sharp object 270 may be packaged in the box apparatus 2000. According to the above process, when the sharp object 270 is disassembled, the application apparatus 1000 is operated to pick up the first upper body part 860a, the sensor 820 and the sharp object 270 in the box apparatus 2000. The application apparatus 1000 and the box apparatus 2000 are moved oppositely, the moving main body 200 returns to a held state under the pushing effect of a loading platform in the box apparatus 2000, that is, the protrusion 236b of the first locking portion 236 is overlapped with the first holding portion 130. The first upper body part 860a is coupled with the accommodating portion 250. The sharp object 270 in the box apparatus 2000 is received and fixed by the sharp object fixing portion 282.

The first upper body part 860a is coupled with the accommodating portion 250, the needle-shaped portion 272 of the sharp object 270 passes through the sensor coupling hole 842, and the implanting part and the extending part of the sensor 820 may be placed in the groove 274 of the sharp object 270. By the application phase described herein, the first upper body part 860a may be applied to the body surface of the host, and the sensor 820 may be partially placed subcutaneously on the host. In some examples, the state in which the first upper body part 860a is coupled with the accommodating portion 250 is a factory state of a split medical instrument suite. That is, when the split medical instrument suite is delivered, the first upper body part 860a and the sensor 820 may be coupled with the accommodating portion 250 in advance and packaged in the housing 10.

Although the present invention has been specifically described above in conjunction with the accompanying drawings and embodiments, it is to be understood that the above description does not limit the present invention in any form. Those skilled in the art may transform and change the present invention as needed without deviating from the essential spirit and scope of the present invention, and these deformations and changes fall within the scope of the present invention.

## Claims

1. An application apparatus for applying a medical instrument to a host, **characterized by** including a housing, an auxiliary mechanism and a first driving mechanism, the housing includes a first holding portion, a proximate end close to the host during operation and a distal end far away from the host, the auxiliary mechanism includes a moving main body releasably held on the first holding portion and configured to move relative to the housing when released, an accommodating portion provided on the moving main body and configured to be combined with the medical instrument, and a piercing member provided on the moving main body;
the first driving mechanism is configured to apply a force to the moving main body in a mode of facing the proximate end; and the first driving mechanism drives the moving main body to move towards the proximate end when the moving main body is released so as to push the medical instrument combined with the accommodating portion to a body surface of the host and to at least partially place the medical instrument subcutaneously on the host.

2. The application apparatus according to claim 1, **characterized in that** the medical instrument includes a sensor which may be partially placed subcutaneously on the host and an application portion coupled with the sensor, the application portion has a first upper body part physically connected with the sensor and applied to the body surface of the host, and a second upper body part electrically connected with the sensor and detachably assembled with the first upper body part.

3. The application apparatus according to claim 2, **characterized in that** the accommodating portion is detachably coupled to the first upper body part.

4. The application apparatus according to claim 2, **characterized in that** the piercing member comprises a sharp object passing through the accommodating portion and configured to at least partially store the sensor, and a support seat releasably supports the sharp object.

5. The application apparatus according to claim 4, **characterized by** further including a disassembling mechanism for separating the sharp object from the support seat.

6. The application apparatus according to claim 1, **characterized in that** the moving main body includes a first locking portion configured to be releasably interlocked with the first holding portion.

7. The application apparatus according to claim 6, **characterized by** further including a first triggering mechanism configured to enable the first holding portion to be separated from the first locking portion so as to release the moving main body.

8. The application apparatus according to claim 1, **characterized in that** the auxiliary mechanism further includes a second holding portion provided on the moving main body, and the piercing member is releasably held on the second holding portion and is configured to move relative to the accommodating portion when released.

9. The application apparatus according to claim 8, **characterized in that** the piercing member includes a second locking portion configured to be releasably interlocked with the second holding portion.

10. The application apparatus according to claim 8, **characterized in that** the application apparatus further includes a second driving mechanism, and the second driving mechanism is configured to apply a force to the piercing member in a mode of facing the distal end.
